# EUROPEAN PATENT SPECIFICATION

(11) **EP 0 785 949 B1**
(45) Date of publication and mention of the grant of the patent: **02.05.2003**
(21) Application number: 95936309.4
(22) Date of filing: 12.10.1995
(51) Int. Cl.: C07K 14/50

(54) **METHOD FOR PURIFYING KERATINOCYTE GROWTH FACTORS**
METHODE ZUR REINIGUNG VON KERATINOCYTEN-WACHSTUMSFAKTOREN
PROCEDE POUR PURIFIER DES FACTEURS DE CROISSANCE DES KERATINOCYTES

(30) Priority: 13.10.1994 US 323339; 07.06.1995 US 487830
(43) Date of publication of application: 30.07.1997
(60) Divisional application: 02028624.1
(73) Proprietor: Amgen Inc., Thousand Oaks, CA 91320-1799 (US)
(72) Inventor: HSU, Eric, W., Thousand Oaks, CA 91320-1789 (US); KENNEY, William, C., Thousand Oaks, CA 91320-1789 (US); TRESSEL, Tim, Thousand Oaks, CA 91320-1789 (US)
(74) Representative: Grünecker, Kinkeldey, Stockmair & Schwanhäusser Anwaltssozietät
(86) International application number: US9513099
(87) International publication number: WO96011952

(56) References cited:
- WO-A-90/08771
- FEBS LETTERS, vol. 328, no. 1,2, - August 1993 AMSTERDAM NL, pages 17-20, M.SUZUKI E A 'Splee-derived growth factor, SDGF-3, is identified as keratinocyte growth factor (KGF)'
- JOURNAL OF BIOLOGICAL CHEMISTRY, vol. 268, no. 4, 5 February 1993 MD US, pages 2984-2988, D.RON ET AL 'Expression of biologically active recombinant KGF' cited in the application

### SEQUENCE LISTING

### (1) GENERAL INFORMATION:

(i) APPLICANT: Amgen Inc.
(ii) TITLE OF INVENTION: Method for Purifying Keratinocyte
   Growth Factors
(iii) NUMBER OF SEQUENCES: 52
(iv) CORRESPONDENCE ADDRESS:
   (A) ADDRESSEE: Amgen Inc.
   (B) STREET: 1840 DeHavilland Drive
   (C) CITY: Thousand Oaks
   (D) STATE: California
   (E) COUNTRY: U.S.A.
   (F) ZIP: 91320-1789
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER: US 08/487,830
   (B) FILING DATE:
   (C) CLASSIFICATION: not yet known

### (2) INFORMATION FOR SEQ ID NO:1:

(i) SEQUENCE CHARACTERISTICS:
   (A) LENGTH: 862 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:1:
   CAATCTACAA TTCACAGATA GGAAGAGGTC AATGACCTAG GAGTAACAAT CAACTCAAGA     60
   TTCATTTTCA TTATGTTATT CATGAACACC CGGAGCACTA CACTATAATG CACAAATGGA    120
   TACTGACATG GATCCTGCCA ACTTTGCTCT ACAGATCATG CTTTCACATT ATCTGTCTAG    180
   TGGGTACTAT ATCTTTAGCT TGCAATGACA TGACTCCAGA GCAAATGGCT ACAAATGTGA    240
   ACTGTTCCAG CCCTGAGCGA CACACAAGAA GTTATGATTA CATGGAAGGA GGGGATATAA    300
   GAGTGAGAAG ACTCTTCTGT CGAACACAGT GGTACCTGAG GATCGATAAA AGAGGCAAAG      360
   TAAAAGGGAC CCAAGAGATG AAGAATAATT ACAATATCAT GGAAATCAGG ACAGTGGCAG      420
   TTGGAATTGT GGCAATCAAA GGGGTGGAAA GTGAATTCTA TCTTGCAATG AACAAGGAAG      480
   GAAAACTCTA TGCAAAGAAA GAATGCAATG AAGATTGTAA CTTCAAAGAA CTAATTCTGG      540
   AAAACCATTA CAACACATAT GCATCAGCTA AATGGACACA CAACGGAGGG GAAATGTTTG      600
   TTGCCTTAAA TCAAAAGGGG ATTCCTGTAA GAGGAAAAAA AACGAAGAAA GAACAAAAAA      660
   CAGCCCACTT TCTTCCTATG GCAATAACTT AATTGCATAT GGTATATAAA GAACCCAGTT      720
   CCAGCAGGGA GATTTCTTTA AGTGGACTGT TTTCTTTCTT CTCAAAATTT TCTTTCCTTT      780
   TATTTTTTAG TAATCAAGAA AGGCTGGAAA AACTACTGAA AAACTGATCA AGCTGGACTT      840
   GTGCATTTAT GTTTGTTTTA AG                                               862

### (2) INFORMATION FOR SEQ ID NO:2:

(i) SEQUENCE CHARACTERISTICS:
   (A) LENGTH: 194 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:2:
   Met His Lys Trp Ile Leu Thr Trp Ile Leu Pro Thr Leu Leu Tyr Arg
   1               5                   10                  15
   Ser Cys Phe His Ile Ile Cys Leu Val Gly Thr Ile Ser Leu Ala Cys
               20                  25                  30
   Asn Asp Met Thr Pro Glu Gln Met Ala Thr Asn Val Asn Cys Ser Ser
           35                  40                  45
   Pro Glu Arg His Thr Arg Ser Tyr Asp Tyr Met Glu Gly Gly Asp Ile
       50                  55                  60
   Arg Val Arg Arg Leu Phe Cys Arg Thr Gln Trp Tyr Leu Arg Ile Asp
   65                  70                  75                  80
   Lys Arg Gly Lys Val Lys Gly Thr Gln Glu Met Lys Asn Asn Tyr Asn
                   85                  90                  95
   Ile Met Glu Ile Arg Thr Val Ala Val Gly Ile Val Ala Ile Lys Gly
               100                 105                 110
   Val Glu Ser Glu Phe Tyr Leu Ala Met Asn Lys Glu Gly Lys Leu Tyr
           115                 120                 125
   Ala Lys Lys Glu Cys Asn Glu Asp Cys Asn Phe Lys Glu Leu Ile Leu
       130                 135                 140
   Glu Asn His Tyr Asn Thr Tyr Ala Ser Ala Lys Trp Thr His Asn Gly
   145                 150                 155                 160
   Gly Glu Met Phe Val Ala Leu Asn Gln Lys Gly Ile Pro Val Arg Gly
                   165                 170                 175
   Lys Lys Thr Lys Lys Glu Gln Lys Thr Ala His Phe Leu Pro Met Ala
               180                 185                 190
   Ile Thr

### (2) INFORMATION FOR SEQ ID NO:3:

(i) SEQUENCE CHARACTERISTICS:
   (A) LENGTH: 595 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:3:
   ATCGATTTGA TTCTAGAAGG AGGAATAACA TATGAAAAAG CGCGCACGTG CTATCGCCAT    60
   TGCTGTGGCT CTGGCAGGTT TCGCAACTAG TGCACACGCG TGCAATGACA TGACTCCAGA   120
   GCAAATGGCT ACAAATGTGA ACTGTTCCAG CCCTGAGCGA CACACAAGAA GTTATGATTA   180
   CATGGAAGGA GGGGATATAA GAGTGAGAAG ACTCTTCTGT CGAACACAGT GGTACCTGAG   240
   GATCGATAAA AGAGGCAAAG TAAAAGGGAC CCAAGAGATG AAGAATAATT ACAATATCAT   300
   GGAAATCAGG ACAGTGGCAG TTGGAATTGT GGCAATCAAA GGGGTGGAAA GTGAATTCTA   360
   TCTTGCAATG AACAAGGAAG GAAAACTCTA TGCAAAGAAA GAATGCAATG AAGATTGTAA   420
   CTTCAAAGAA CTAATTCTGG AAAACCATTA CAACACATAT GCATCAGCTA AATGGACACA   480
   CAACGGAGGG GAAATGTTTG TTGCCTTAAA TCAAAAGGGG ATTCCTGTAA GAGGAAAAAA   540
   AACGAAGAAA GAACAAAAAA CAGCCCACTT TCTTCCTATG GCAATAACTT AATAG        595

### (2) INFORMATION FOR SEQ ID NO:4:

(i) SEQUENCE CHARACTERISTICS:
   (A) LENGTH: 186 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:4:
   Met Lys Lys Arg Ala Arg Ala Ile Ala Ile Ala Val Ala Leu Ala Gly
   1               5                   10                  15
   Phe Ala Thr Ser Ala His Ala Cys Asn Asp Met Thr Pro Glu Gln Met
               20                  25                  30
   Ala Thr Asn Val Asn Cys Ser Ser Pro Glu Arg His Thr Arg Ser Tyr
           35                  40                  45
   Asp Tyr Met Glu Gly Gly Asp Ile Arg Val Arg Arg Leu Phe Cys Arg
       50                  55                  60
   Thr Gln Trp Tyr Leu Arg Ile Asp Lys Arg Gly Lys Val Lys Gly Thr
   65                  70                  75                  80
   Gln Glu Met Lys Asn Asn Tyr Asn Ile Met Glu Ile Arg Thr Val Ala
                   85                  90                  95
   Val Gly Ile Val Ala Ile Lys Gly Val Glu Ser Glu Phe Tyr Leu Ala
               100                 105                 110
   Met Asn Lys Glu Gly Lys Leu Tyr Ala Lys Lys Glu Cys Asn Glu Asp
           115                 120                 125
   Cys Asn Phe Lys Glu Leu Ile Leu Glu Asn His Tyr Asn Thr Tyr Ala
       130                 135                 140
   Ser Ala Lys Trp Thr His Asn Gly Gly Glu Met Phe Val Ala Leu Asn
   145                 150                 155                 160
   Gln Lys Gly Ile Pro Val Arg Gly Lys Lys Thr Lys Lys Glu Gln Lys
                   165                 170                 175
   Thr Ala His Phe Leu Pro Met Ala Ile Thr
               180                 185

### (2) INFORMATION FOR SEQ ID NO:5:

(i) SEQUENCE CHARACTERISTICS:
   (A) LENGTH: 499 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:5:
   TATGTGCAAT GACATGACTC CAGAGCAAAT GGCTACAAAT GTGAACTGTT CCAGCCCTGA    60
   GCGACACACA AGAAGTTATG ATTACATGGA AGGAGGGGAT ATAAGAGTGA GAAGACTCTT   120
   CTGTCGAACA CAGTGGTACC TGAGGATCGA TAAAAGAGGC AAAGTAAAAG GGACCCAAGA   180
   GATGAAGAAT AATTACAATA TCATGGAAAT CAGGACAGTG GCAGTTGGAA TTGTGGCAAT      240
   CAAAGGGGTG GAAAGTGAAT TCTATCTTGC AATGAACAAG GAAGGAAAAC TCTATGCAAA      300
   GAAAGAATGC AATGAAGATT GTAACTTCAA AGAACTAATT CTGGAAAACC ATTACAACAC      360
   ATATGCATCA GCTAAATGGA CACACAACGG AGGGGAAATG TTTGTTGCCT TAAATCAAAA      420
   GGGGATTCCT GTAAGAGGAA AAAAAACGAA GAAAGAACAA AAAACAGCCC ACTTTCTTCC      480
   TATGGCAATA ACTTAATAG                                                  499

### (2) INFORMATION FOR SEQ ID NO:6:

(i) SEQUENCE CHARACTERISTICS:
   (A) LENGTH: 164 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:6:
   Met Cys Asn Asp Met Thr Pro Glu Gln Met Ala Thr Asn Val Asn Cys
   1               5                   10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Arg Gly Lys Lys Thr Lys Lys Glu Gln Lys Thr Ala His Phe Leu Pro
   145                 150                 155                 160
   Met Ala Ile Thr

### (2) INFORMATION FOR SEQ ID NO:7:

(i) SEQUENCE CHARACTERISTICS:
   (A) LENGTH: 25 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:7:
   CAATGACCTA GGAGTAACAA TCAAC                    25

### (2) INFORMATION FOR SEQ ID NO:8:

(i) SEQUENCE CHARACTERISTICS:
   (A) LENGTH: 26 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:8:
   AAAACAAACA TAAATGCACA AGTCCA 26

### (2) INFORMATION FOR SEQ ID NO:9:

(i) SEQUENCE CHARACTERISTICS:
   (A) LENGTH: 26 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:9:
   ACAACGCGTG CAATGACATG ACTCCA 26

### (2) INFORMATION FOR SEQ ID NO:10:

(i) SEQUENCE CHARACTERISTICS:
   (A) LENGTH: 31 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:10:
   ACAGGATCCT ATTAAGTTAT TGCCATAGGA A 31

### (2) INFORMATION FOR SEQ ID NO:11:

(i) SEQUENCE CHARACTERISTICS:
   (A) LENGTH: 27 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:11:
   ACACATATGT GCAATGACAT GACTCCA 27

### (2) INFORMATION FOR SEQ ID NO:12:

(i) SEQUENCE CHARACTERISTICS:
   (A) LENGTH: 37 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:12:
   CTGCGTATCG ACAAACGCGG CAAAGTCAAG GGCACCC 37

### (2) INFORMATION FOR SEQ ID NO:13:

(i) SEQUENCE CHARACTERISTICS:
   (A) LENGTH: 44 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:13:
   AAGAGATGAA AAACAACTAC AATATTATGG AAATCCGTAC TGTT 44

### (2) INFORMATION FOR SEQ ID NO:14:

(i) SEQUENCE CHARACTERISTICS:
   (A) LENGTH: 37 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:14:
   GCTGTTGGTA TCGTTGCAAT CAAAGGTGTT GAATCTG 37

### (2) INFORMATION FOR SEQ ID NO:15:

(i) SEQUENCE CHARACTERISTICS:
   (A) LENGTH: 45 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:15:
   TCTTGGGTGC CCTTGACTTT GCCGCGTTTG TCGATACGCA GGTAC 45

### (2) INFORMATION FOR SEQ ID NO:16:

(i) SEQUENCE CHARACTERISTICS:
   (A) LENGTH: 45 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:16:
   ACAGCAACAG TACGGATTTC CATAATATTG TAGTTGTTTT TCATC 45

### (2) INFORMATION FOR SEQ ID NO:17:

(i) SEQUENCE CHARACTERISTICS:
   (A) LENGTH: 36 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:17:
   AATTCAGATT CAACACCTTT GATTGCAACG ATACCA 36

### (2) INFORMATION FOR SEQ ID NO:18:

(i) SEQUENCE CHARACTERISTICS:
   (A) LENGTH: 20 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:18:
   AGTTTTGATC TAGAAGGAGG 20

### (2) INFORMATION FOR SEQ ID NO:19:

(i) SEQUENCE CHARACTERISTICS:
   (A) LENGTH: 20 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:19:
   TCAAAACTGG ATCCTATTAA 20

### (2) INFORMATION FOR SEQ ID NO:20:

(i) SEQUENCE CHARACTERISTICS:
   (A) LENGTH: 91 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:20:
   AGTTTTGATC TAGAAGGAGG AATAACATAT GTGCAACGAC ATGACTCCGG AACAGATGGC 60
   TACCAACGTT AACTGCTCCA GCCCGGAACG T 91

### (2) INFORMATION FOR SEQ ID NO:21:

(i) SEQUENCE CHARACTERISTICS:
   (A) LENGTH: 90 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:21:
   CACACCCGTA GCTACGACTA CATGGAAGGT GGTGACATCC GTGTTCGTCG TCTGTTCTGC    60
   CGTACCCAGT GGTACCTGCG TATCGACAAA                                     90

### (2) INFORMATION FOR SEQ ID NO:22:

(i) SEQUENCE CHARACTERISTICS:
   (A) LENGTH: 90 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:22:
   CGTGGTAAAG TTAAAGGTAC CCAGGAAATG AAAAACAACT ACAACATCAT GGAAATCCGT    60
   ACTGTTGCTG TTGGTATCGT TGCAATCAAA                                     90

### (2) INFORMATION FOR SEQ ID NO:23:

(i) SEQUENCE CHARACTERISTICS:
   (A) LENGTH: 90 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:23:
   GGTGTTGAAT CTGAATTCTA CCTGGCAATG AACAAAGAAG GTAAACTGTA CGCAAAAAAA 60
   GAATGCAACG AAGACTGCAA CTTCAAAGAA 90

### (2) INFORMATION FOR SEQ ID NO:24:

(i) SEQUENCE CHARACTERISTICS:
   (A) LENGTH: 90 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:24:
   CTGATCCTGG AAAACCACTA CAACACCTAC GCATCTGCTA AATGGACCCA CAACGGTGGT 60
   GAAATGTTCG TTGCTCTGAA CCAGAAAGGT 90

### (2) INFORMATION FOR SEQ ID NO:25:

(i) SEQUENCE CHARACTERISTICS:
   (A) LENGTH: 88 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:25:
   ATCCCGGTTC GTGGTAAAAA AACCAAAAAA GAACAGAAAA CCGCTCACTT CCTGCCGATG 60
   GCAATCACTT AATAGGATCC AGTTTTGA 88

### (2) INFORMATION FOR SEQ ID NO:26:

(i) SEQUENCE CHARACTERISTICS:
   (A) LENGTH: 20 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:26:
   TACGGGTGTG ACGTTCCGGG 20

### (2) INFORMATION FOR SEQ ID NO:27:

(i) SEQUENCE CHARACTERISTICS:
   (A) LENGTH: 20 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:27:
   CTTTACCACG TTTGTCGATA 20

### (2) INFORMATION FOR SEQ ID NO:28:

(i) SEQUENCE CHARACTERISTICS:
   (A) LENGTH: 20 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:28:
   ATTCAACACC TTTGATTGCA 20

### (2) INFORMATION FOR SEQ ID NO:29:

(i) SEQUENCE CHARACTERISTICS:
   (A) LENGTH: 20 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:29:
   CCAGGATCAG TTCTTTGAAG 20

### (2) INFORMATION FOR SEQ ID NO:30:

(i) SEQUENCE CHARACTERISTICS:
   (A) LENGTH: 20 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:30:
   GAACCGGGAT ACCTTTCTGG 20

### (2) INFORMATION FOR SEQ ID NO:31:

(i) SEQUENCE CHARACTERISTICS:
   (A) LENGTH: 495 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:31:
   ATGTCTAATG ATATGACTCC GGAACAGATG GCTACCAACG TTAACTCCTC CTCCCCGGAA 60
   CGTCACACGC GTTCCTACGA CTACATGGAA GGTGGTGACA TCCGCGTACG TCGTCTGTTC 120
   TGCCGTACCC AGTGGTACCT GCGTATCGAC AAACGCGGCA AAGTCAAGGG CACCCAAGAG 180
   ATGAAAAACA ACTACAATAT TATGGAAATC CGTACTGTTG CTGTTGGTAT CGTTGCAATC 240
   AAAGGTGTTG AATCTGAATT CTACCTGGCA ATGAACAAAG AAGGTAAACT GTACGCAAAA 300
   AAAGAATGCA ACGAAGACTG CAACTTCAAA GAACTGATCC TGGAAAACCA CTACAACACC 360
   TACGCATCTG CTAAATGGAC CCACAACGGT GGTGAAATGT TCGTTGCTCT GAACCAGAAA 420
   GGTATCCCGG TTCGTGGTAA AAAAACCAAA AAAGAACAGA AAACCGCTCA CTTCCTGCCG 480
   ATGGCAATCA CTTAA 495

### (2) INFORMATION FOR SEQ ID NO:32:

(i) SEQUENCE CHARACTERISTICS:
   (A) LENGTH: 164 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:32:
   Met Ser Asn Asp Met Thr Pro Glu Gln Met Ala Thr Asn Val Asn Ser
   1               5                   10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Arg Gly Lys Lys Thr Lys Lys Glu Gln Lys Thr Ala His Phe Leu Pro
   145                 150                 155                 160
   Met Ala Ile Thr

### (2) INFORMATION FOR SEQ ID NO:33:

(i) SEQUENCE CHARACTERISTICS:
   (A) LENGTH: 495 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:33:
   ATGTGCAATG ATATGACTCC TGAACAAATG GCTACCAATG TCAACTGTTC CTCTCCGGAG     60
   CGCCACACCC GGAGTTACGA TTACATGGAA GGTGGGGATA TTCGCGTACG TCGTCTGTTC    120
   TGCCGTACCC AGTGGTACCT GCGTATCGAC AAACGCGGCA AAGTCAAGGG CACCCAAGAG    180
   ATGAAAAACA ACTACAATAT TATGGAAATC CGTACTGTTG CTGTTGGTAT CGTTGCAATC    240
   AAAGGTGTTG AATCTGAATT CTATCTTGCA ATGAACAAGG AAGGAAAACT CTATGCAAAG    300
   AAAGAATGCA ATGAAGATTG TAACTTCAAA GAACTAATTC TGGAAAACCA TTACAACACA    360
   TATGCATCTG CTAAATGGAC CCACAACGGT GGTGAAATGT TCGTTGCTCT GAACCAGAAA    420
   GGTATCCCTG TTCAAGGTAA GAAAACCAAG AAAGAACAGA AAACCGCTCA CTTCCTGCCG    480
   ATGGCAATCA CTTAA                                                     495

### (2) INFORMATION FOR SEQ ID NO:34:

(i) SEQUENCE CHARACTERISTICS:
   (A) LENGTH: 164 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:34:
   Met Cys Asn Asp Met Thr Pro Glu Gln Met Ala Thr Asn Val Asn Cys
   1               5                   10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Gln Gly Lys Lys Thr Lys Lys Glu Gln Lys Thr Ala His Phe Leu Pro
   145                 150                 155                 160
   Met Ala Ile Thr

### (2) INFORMATION FOR SEQ ID NO:35:

(i) SEQUENCE CHARACTERISTICS:
   (A) LENGTH: 495 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:35:
   ATGTCTAATG ATATGACTCC GGAACAGATG GCTACCAACG TTAACTCCTC CTCCCCGGAA    60
   CGTCACACGC GTTCCTACGA CTACATGGAA GGTGGTGACA TCCGCGTACG TCGTCTGTTC   120
   TGCCGTACCC AGTGGTACCT GCGTATCGAC AAACGCGGCA AAGTCAAGGG CACCCAAGAG   180
   ATGAAAAACA ACTACAATAT TATGGAAATC CGTACTGTTG CTGTTGGTAT CGTTGCAATC   240
   AAAGGTGTTG AATCTGAATT CTATCTTGCA ATGAACAAGG AAGGAAAACT CTATGCAAAG   300
   AAAGAATGCA ATGAAGATTG TAACTTCAAA GAACTAATTC TGGAAAACCA TTACAACACA   360
   TATGCATCTG CTAAATGGAC CCACAACGGT GGTGAAATGT TCGTTGCTCT GAACCAGAAA   420
   GGTATCCCTG TTCAAGGTAA GAAAACCAAG AAAGAACAGA AAACCGCTCA CTTCCTGCCG   480
   ATGGCAATCA CTTAA                                                    495

### (2) INFORMATION FOR SEQ ID NO:36:

(i) SEQUENCE CHARACTERISTICS:
   (A) LENGTH: 164 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:36:
   Met Ser Asn Asp Met Thr Pro Glu Gln Met Ala Thr Asn Val Asn Ser
   1               5                   10                  15
   Ser Ser Pro Glu Arg His Thr Arg Ser Tyr Asp Tyr Met Glu Gly Gly
               20                  25                  30
   Asp Ile Arg Val Arg Arg Leu Phe Cys Arg Thr Gln Trp Tyr Leu Arg
           35                  40                  45
   Ile Asp Lys Arg Gly Lys Val Lys Gly Thr Gln Glu Met Lys Asn Asn
       50                  55                  60
   Tyr Asn Ile Met Glu Ile Arg Thr Val Ala Val Gly Ile Val Ala Ile
   65                  70                  75                  80
   Lys Gly Val Glu Ser Glu Phe Tyr Leu Ala Met Asn Lys Glu Gly Lys
                   85                  90                  95
   Leu Tyr Ala Lys Lys Glu Cys Asn Glu Asp Cys Asn Phe Lys Glu Leu
               100                 105                 110
   Ile Leu Glu Asn His Tyr Asn Thr Tyr Ala Ser Ala Lys Trp Thr His
           115                 120                 125
   Asn Gly Gly Glu Met Phe Val Ala Leu Asn Gln Lys Gly Ile Pro Val
       130                 135                 140
   Gln Gly Lys Lys Thr Lys Lys Glu Gln Lys Thr Ala His Phe Leu Pro
   145                 150                 155                 160
   Met Ala Ile Thr

### (2) INFORMATION FOR SEQ ID NO:37:

(i) SEQUENCE CHARACTERISTICS:
   (A) LENGTH: 450 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:37:
   ATGTCTTCTC CTGAACGTCA TACGCGTTCC TACGACTACA TGGAAGGTGG TGACATCCGC     60
   GTACGTCGTC TGTTCTGCCG TACCCAGTGG TACCTGCGTA TCGACAAACG CGGCAAAGTC    120
   AAGGGCACCC AAGAGATGAA AAACAACTAC AATATTATGG AAATCCGTAC TGTTGCTGTT    180
   GGTATCGTTG CAATCAAAGG TGTTGAATCT GAATTCTACC TGGCAATGAA CAAAGAAGGT    240
   AAACTGTACG CAAAAAAAGA ATGCAACGAA GACTGCAACT TCAAAGAACT GATCCTGGAA    300
   AACCACTACA ACACCTACGC ATCTGCTAAA TGGACCCACA ACGGTGGTGA AATGTTCGTT    360
   GCTCTGAACC AGAAAGGTAT CCCGGTTCGT GGTAAAAAAA CCAAAAAAGA ACAGAAAACC    420
   GCTCACTTCC TGCCGATGGC AATCACTTAA                                     450

### (2) INFORMATION FOR SEQ ID NO:38:

(i) SEQUENCE CHARACTERISTICS:
   (A) LENGTH: 149 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:38:
   Met Ser Ser Pro Glu Arg His Thr Arg Ser Tyr Asp Tyr Met Glu Gly
   1               5                   10                  15
   Gly Asp Ile Arg Val Arg Arg Leu Phe Cys Arg Thr Gln Trp Tyr Leu
               20                  25                  30
   Arg Ile Asp Lys Arg Gly Lys Val Lys Gly Thr Gln Glu Met Lys Asn
           35                  40                  45
   Asn Tyr Asn Ile Met Glu Ile Arg Thr Val Ala Val Gly Ile Val Ala
       50                  55                  60
   Ile Lys Gly Val Glu Ser Glu Phe Tyr Leu Ala Met Asn Lys Glu Gly
   65                  70                  75                  80
   Lys Leu Tyr Ala Lys Lys Glu Cys Asn Glu Asp Cys Asn Phe Lys Glu
                   85                  90                  95
   Leu Ile Leu Glu Asn His Tyr Asn Thr Tyr Ala Ser Ala Lys Trp Thr
               100                 105                 110
   His Asn Gly Gly Glu Met Phe Val Ala Leu Asn Gln Lys Gly Ile Pro
           115                 120                 125
   Val Arg Gly Lys Lys Thr Lys Lys Glu Gln Lys Thr Ala His Phe Leu
       130                 135                 140
   Pro Met Ala Ile Thr
   145

### (2) INFORMATION FOR SEQ ID NO:39:

(i) SEQUENCE CHARACTERISTICS:
   (A) LENGTH: 426 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:39:
   ATGTCCTACG ACTACATGGA AGGTGGTGAC ATCCGCGTAC GTCGTCTGTT CTGCCGTACC      60
   CAGTGGTACC TGCGTATCGA CAAACGCGGC AAAGTCAAGG GCACCCAAGA GATGAAAAAC     120
   AACTACAATA TTATGGAAAT CCGTACTGTT GCTGTTGGTA TCGTTGCAAT CAAAGGTGTT     180
   GAATCTGAAT TCTACCTGGC AATGAACAAA GAAGGTAAAC TGTACGCAAA AAAAGAATGC     240
   AACGAAGACT GCAACTTCAA AGAACTGATC CTGGAAAACC ACTACAACAC CTACGCATCT     300
   GCTAAATGGA CCCACAACGG TGGTGAAATG TTCGTTGCTC TGAACCAGAA AGGTATCCCG     360
   GTTCGTGGTA AAAAAACCAA AAAAGAACAG AAAACCGCTC ACTTCCTGCC GATGGCAATC     420
   ACTTAA                                                                426

### (2) INFORMATION FOR SEQ ID NO:40:

(i) SEQUENCE CHARACTERISTICS:
   (A) LENGTH: 141 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:40:
   Met Ser Tyr Asp Tyr Met Glu Gly Gly Asp Ile Arg Val Arg Arg Leu
   1               5                   10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Arg Gly Lys Lys Thr Lys Lys
           115                 120                 125
   Glu Gln Lys Thr Ala His Phe Leu Pro Met Ala Ile Thr
       130                 135                 140

### (2) INFORMATION FOR SEQ ID NO:41:

(i) SEQUENCE CHARACTERISTICS:
   (A) LENGTH: 426 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:41:
   ATGTCCTACG ACTACATGGA AGGTGGTGAC ATCCGCGTAC GTCGTCTGTT CTGCCGTACC    60
   CAGTGGTACC TGCGTATCGA CAAACGCGGC AAAGTCAAGG GCACCCAAGA GATGAAAAAC   120
   AACTACAATA TTATGGAAAT CCGTACTGTT GCTGTTGGTA TCGTTGCAAT CAAAGGTGTT   180
   GAATCTGAAT TCTATCTTGC AATGAACAAG GAAGGAAAAC TCTATGCAAA GAAAGAATGC   240
   AATGAAGATT GTAACTTCAA AGAACTAATT CTGGAAAACC ATTACAACAC ATATGCATCT   300
   GCTAAATGGA CCCACAACGG TGGTGAAATG TTCGTTGCTC TGAACCAGAA AGGTATCCCT   360
   GTTCAAGGTA AGAAAACCAA GAAAGAACAG AAAACCGCTC ACTTCCTGCC GATGGCAATC   420
   ACTTAA                                                              426

### (2) INFORMATION FOR SEQ ID NO:42:

(i) SEQUENCE CHARACTERISTICS:
   (A) LENGTH: 141 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:42:
   Met Ser Tyr Asp Tyr Met Glu Gly Gly Asp Ile Arg Val Arg Arg Leu
   1               5                   10                  15
   Phe Cys Arg Thr Gln Trp Tyr Leu Arg Ile Asp Lys Arg Gly Lys Val
               20                  25                  30
   Lys Gly Thr Gln Glu Met Lys Asn Asn Tyr Asn Ile Met Glu Ile Arg
           35                  40                  45
   Thr Val Ala Val Gly Ile Val Ala Ile Lys Gly Val Glu Ser Glu Phe
       50                  55                  60
   Tyr Leu Ala Met Asn Lys Glu Gly Lys Leu Tyr Ala Lys Lys Glu Cys
   65                  70                  75                  80
   Asn Glu Asp Cys Asn Phe Lys Glu Leu Ile Leu Glu Asn His Tyr Asn
                   85                  90                  95
   Thr Tyr Ala Ser Ala Lys Trp Thr His Asn Gly Gly Glu Met Phe Val
               100                 105                 110
   Ala Leu Asn Gln Lys Gly Ile Pro Val Gln Gly Lys Lys Thr Lys Lys
           115                 120                 125
   Glu Gln Lys Thr Ala His Phe Leu Pro Met Ala Ile Thr
       130                 135                 140

### (2) INFORMATION FOR SEQ ID NO:43:

(i) SEQUENCE CHARACTERISTICS:
   (A) LENGTH: 24 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:43:
   GAGCTCACTA GTGTCGACCT GCAG                                        24

### (2) INFORMATION FOR SEQ ID NO:44:

(i) SEQUENCE CHARACTERISTICS:
   (A) LENGTH: 24 base pairs
   (B) TYPE: nucleic acid
   (C) STRANDEDNESS: unknown
   (D) TOPOLOGY: unknown
(ii) MOLECULE TYPE: cDNA
(ix) FEATURE:
   (A) NAME/KEY: -
   (B) LOCATION: complement (1..24)
(xi) SEQUENCE DESCRIPTION: SEQ ID NO:44:
   CTGCAGGTCG ACACTAGTGA GCTC 24

### (2) INFORMATION FOR SEQ ID NO:45:

(i) SEQUENCE CHARACTERISTICS:
   (A) LENGTH: 18 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:45:
   CAATCTACAA TTCACAGA 18

### (2) INFORMATION FOR SEQ ID NO:46:

(i) SEQUENCE CHARACTERISTICS:
   (A) LENGTH: 18 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:46:
   TTAAGTTATT GCCATAGG 18

### (2) INFORMATION FOR SEQ ID NO:47:

(i) SEQUENCE CHARACTERISTICS:
   (A) LENGTH: 29 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:47:
   AACAAAGCTT CTACAATTCA CAGATAGGA 29

### (2) INFORMATION FOR SEQ ID NO:48:

(i) SEQUENCE CHARACTERISTICS:
   (A) LENGTH: 27 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:48:
   AACAAGATCT TAAGTTATTG CCATAGG 27

### (2) INFORMATION FOR SEQ ID NO:49:

(i) SEQUENCE CHARACTERISTICS:
   (A) LENGTH: 38 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:49:
   CGGTCTAGAC CACCATGCAC AAATGGATAC TGACATGG 38

### (2) INFORMATION FOR SEQ ID NO:50:

(i) SEQUENCE CHARACTERISTICS:
   (A) LENGTH: 33 base pairs
   (B) TYPE: nucleic acid
   (C) STRANDEDNESS: unknown
   (D) TOPOLOGY: unknown
(ii) MOLECULE TYPE: cDNA
(xi) SEQUENCE DESCRIPTION: SEQ ID NO:50:
   GCCGTCGACC TATTAAGTTA TTGCCATAGG AAG                                    33

### (2) INFORMATION FOR SEQ ID NO:51:

(i) SEQUENCE CHARACTERISTICS:
   (A) LENGTH: 16 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:51:
   Xaa Asn Asp Met Thr Pro Glu Gln Met Ala Thr Asn Val Xaa Xaa Ser
   1               5                   10                  15

### (2) INFORMATION FOR SEQ ID NO:52:

(i) SEQUENCE CHARACTERISTICS:
   (A) LENGTH: 12 amino acids
   (B) TYPE: amino acid
   (C) STRANDEDNESS: unknown
   (D) TOPOLOGY: unknown
(ii) MOLECULE TYPE: protein
(xi) SEQUENCE DESCRIPTION: SEQ ID NO:52:
   Ser Tyr Asp Tyr Met Glu Gly Gly Asp Ile Arg Val
   1               5                   10

## Description

### Field of the Invention

The present invention relates to the field of protein purification. Specifically, the present invention relates to the field of purifying keratinocyte growth factors.

### Background of the Invention

Polypeptide growth factors are important mediators of intercellular communication (Rubin *et al.* (1989), *Proc. Natl. Acad. Sci. USA,* 86:802-806). These molecules are generally released by one cell type and act to influence proliferation of other cell types.

One family of growth factors is the fibroblast growth factors (FGF). There are currently eight known FGF family members which share a relatedness among primary structures: basic fibroblast growth factor, bFGF (Abraham *et al.* (1986); *EMBO J.,* 5:2523-2528); acidic fibroblast growth factor, aFGF (Jaye *et al.* (1986), *Science,* 233:541-545); int-2 gene product, int-2 (Dickson & Peters (1987), *Nature,* 326:833); hst/kFGF (Delli-Bovi *et al.* (1987), *Cell,* 50:729-737, and Yoshida *et al.* (1987), *Proc. Natl*. *Acad. Sci. USA,* 84:7305-7309); FGF-5 (Zhan *et al*. (1988), *Mol. Cell. Biol.,* 8:3487-3495); FGF-6 (Marics *et al.* (1989), *Oncogene,* 4:335-340); keratinocyte growth factor (Finch et *al.* (1989), *Science,* 24:752-755; Rubin *et al.* (1989), *Proc. Natl. Acad. Sci. USA,* 86:802-806; Ron *et al.* (1993), The *Journal of Biological Chemistry,* 268(4):2984-2988; and Yan et *al.* (1991), *In Vitro Cell. Dev. Biol.,* 27A:437-438); and hisactophilin (Habazzettl *et al.* (1992), *Nature,* 359:855-858).

Among the FGF family of proteins, keratinocyte growth factor (KGF) is a unique effector of non-fibroblast epithelial (particularly keratinocyte) cell proliferation derived from mesenchymal tissues. The term "native KGF" refers to a natural human (hKGF) or recombinant (rKGF) polypeptide (with or without a signal sequence) as depicted by the amino acid sequence presented in SEQ ID NO:2 or an allelic variant thereof. [Unless otherwise indicated, amino acid numbering for molecules described herein shall correspond to that presented for the mature form of the native molecule (i.e., minus the signal sequence), as depicted by amino acids 32 to 194 of SEQ ID NO:2.]

Native KGF may be isolated from natural sources. For example, hKGF can be isolated from medium conditioned by an embryonic lung fibroblast cell line (Rubin *et al.* (1989), *supra*). Three chromatographic steps, namely heparin-Sepharose™ (Pharmacia, Piscataway, NJ) affinity chromatography, HPLC gel filtration, and reverse-phase HPLC, were used to obtain a purified hKGF preparation. Approximately 6 mg of hKGF were recovered from 10 liters of conditioned medium. These chromatographic steps only recovered 0.8% total hKGF based upon a mitogenic activity assay. A further example teaches the use of another chromatographic step using heparin-Sepharose™ affinity and Mono-S™ ionexchange chromatography (Pharmacia, Piscataway, NJ) for isolation of rKGF produced in bacteria (Ron et al. (1993), *Journal of Biological Chemistry,* 268:2984-2988).

Suzuki, M. et al., (FEBS Vol. 328 No. 1, 2 (1993), 17-20) teaches the partial purification of a heparin-binding mitogenic factor SDGF-3 for rat hepatocytes from bovine spleen by a combination of heparin affinity, cation-exchange and gel-filtration/chromatography. The authors propose the identity of said isolated factor and the keratinocyte growth factor (KGF) due to a number of similar properties and activities. However, there is no clear and unambiguous evidence for the identity of SDGF-3 and KGF due to the considerable differences in size and the fact that the addition of anti-KGF monoclonal antibody does not completely abrogate the growth factor activity of SDGF-3.

The properties of keratinocyte growth factors suggest a potential for the application thereof as a drug for promoting specific stimulation of epithelial cell growth. It therefore would be desirable to develop a method or methods for obtaining relatively high levels of homogeneous keratinocyte growth factors to provide sufficient quantities of material for comprehensive *in vitro* and *in vivo* biological evaluation and for a potential therapeutic application.

It is the object of this invention to provide a novel method for the purification of keratinocyte growth factors.

### Summary of the Invention

The present invention is directed to a method for purifying a keratinocyte growth factor (KGF), the method comprising the following steps:
a) obtaining a solution comprising the KGF;
b) loading the solution of step (a) onto a cation exchange resin;
c) eluting the KGF in an eluate solution from the cation exchange resin;
d) passing the eluate solution from step (c) through either (i) a molecular weight exclusion matrix, or (ii) a hydrophobic interaction chromatography matrix; and
e) recovering the KGF, with the proviso that said method does not comprise a heparin affinity chromatography step.

Generally, the cation exchange chromatography step of the method of the present invention may be conducted with any suitable buffer (e.g., phosphate buffer saline, sodium acetate or tris-HCL) at a pH of preferably between about 6.8-7.5. Suitable columns for use in this step include carboxymethyl cellulose, carboxymethyl agarose and sulfated agarose and cellulose columns (e.g.,columns of S-Sepharose Fast Flow™ resin, Mono-S™ resin and CM-cellulose™ resin, commercially available from Pharmacia, Piscataway, NJ). The flow rate will be variable depending upon the column size.

The gel filtration step of the method may be conducted in any suitable buffer (e.g., phosphate buffer saline) at a pH of preferably between about 7.0 and 7.5. Suitable columns for use in this step include agarose-based, acrylamide-based, silica-based or polymer-based size-exclusion columns (e.g., columns of Sephadex G-75™ resin and Superdex-75™ resin, commercially available from Pharmacia).

In a particularly preferred embodiment of the method, involving a hydrophobic interaction chromatography, free sulfhydryl groups may be oxidized prior to the hydrophobic interaction step, discussed below. Any manner of oxidation may be employed. For example, the protein may be exposed to atmospheric oxygen for a suitable period of time. Alternatively, various oxidation procedures may be employed. One such procedure is particularly suited for keratinocyte growth factors wherein one or more cysteine residues, as compared to the native KGF molecule, are deleted or replaced. In this procedure an oxidizing agent (e.g., cystamine dihydrochloride or another appropriate oxidizing agent, for instance, cystine, oxidized glutathione or divalent copper) may be added to a final concentration, adjusting the pH to preferably between about 7-9.5, with pH 9.0 ± 0.3 being more preferred when using cystamine dihydrochloride), and holding the temperature at preferably between about 10-30°C, for an appropriate period. The second procedure may be used for oxidizing native KGF and other keratinocyte growth factors with comparable patterns of cysteine residues. In this procedure, oxidation may be accomplished by adding an appropriate amount of an ionic strength modifier (e.g., (NH₄)₂SO₄), adjusting the pH to preferably between about 7.5-9.5, and holding the temperature at preferably between about 23 ± 5°C for an appropriate period.

The hydrophobic interaction step may be conducted by using any suitable buffer (e.g., sodium phosphate) at a pH of preferably between about 6.0-8.0, more preferably about 7.0, and by eluting with a decreasing linear (NH₄)₂SO₄ gradient ranging from 2-0 M. Suitable columns for use in this step include alkyl or phenyl substituted resins (e.g., a column of Butyl-650M Toyopearl™ resin, commercially available from Tosohaas, Inc., Montgomeryville, PA and columns of phenyl Sepharose™ resin and phenyl Superose™ resin, commercially available from Pharmacia).

The process of the present invention may be used to purify KGF. Thus, it should be understood that the terms "keratinocyte growth factor" and "KGF" as employed in this description are intended to include, and to mean interchangeably unless otherwise indicated, native KGF and KGF analog proteins (or "muteins") characterized by a peptide sequence substantially the same as the peptide sequence of native KGF and by retaining some or all of the biological activity of native KGF, particularly non-fibroblast epithelial cell proliferation (e.g., exhibiting at least about 500-fold greater stimulation of BALB/MK keratinocyte cells than that of NIH/3T3 fibroblast cells, and at least about 50-fold greater stimulation of BALB/MK keratinocyte cells than for BS/589 epithelial cells or for CCl208 epithelial cells, as determined by H-thymidine incorporation). By "characterized by a peptide sequence substantially the same as the peptide sequence of native KGF" is meant a peptide sequence which is encoded by a DNA sequence capable of hybridizing to nucleotides 201 to 684 of SEQ ID NO:1, preferably under stringent hybridization conditions.

The determination of a corresponding amino acid position between two amino acid sequences may be determined by aligning the two sequences to maximize matches of residues including shifting the amino and/or carboxyl terminus, introducing gaps as required and/or deleting residues present as inserts in the candidate. Database searches, sequence analysis and manipulations may be performed using one of the well-known and routinely used sequence homology/identity scanning algorithm programs (e.g., Pearson and Lipman (1988), *Proc. Natl. Acad. Sci. U.S.A.,* 85:2444-2448; Altschul *et al.* (1990), *J. Mol. Biol.,* 215:403-410; Lipman and Pearson (1985), *Science,* 222:1435 or Devereux *et al.* (1984), *Nuc. Acids Res.,* 12:387-395).

Stringent conditions, in the hybridization context, will be stringent combined conditions of salt, temperature, organic solvents and other parameters typically controlled in hybridization reactions. Exemplary stringent hybridization conditions are hybridization in 4 X SSC at 62-67° C., followed by washing in 0.1 X SSC at 62-67° C. for approximately an hour. Alternatively, exemplary stringent hybridization conditions are hybridization in 45-55% formamide, 4 X SSC at 40-45°C. [*See*, T. Maniatis et. al., *Molecular Cloning* (A Laboratory Manual); Cold Spring Harbor Laboratory (1982), pages 387 to 389].

Thus, the proteins include allelic variations, or deletion(s), substitution(s) or insertion(s) of amino acids, including fragments, chimeric or hybrid molecules of native KGF. One example of KGF includes proteins having residues corresponding to Cys³² and Cys⁴⁶ of SEQ ID NO:2 replaced or deleted, with the resultant molecule having improved stability as compared with the parent molecule (as taught in commonly owned U.S.S.N. 08/487,825, filed on July 7, 1995). Another example of KGF includes charge-change polypeptides wherein one or more of amino acid residues 41-154 of native KGF (preferably residues Arg⁴¹, Gln⁴³, Lys⁵⁵, Lys⁹⁵, Lys¹²⁸, Asn¹³⁷, Gln¹³⁸, Lys¹³⁹, Arg¹⁴⁴, Lys¹⁴⁷, Gln¹⁵², Lys¹⁵³ or Thr¹⁵⁴) are deleted or substituted with a neutral residue or negatively charged residue selected to effect a protein with a reduced positive charge (as taught in commonly owned U.S.S.N. 08/323,337, filed on October 13, 1994). A still further example of KGF includes proteins generated by substituting at least one amino acid having a higher loop-forming potential for at least one amino acid within a loop-forming region of Asn¹¹⁵-His¹¹⁶-Tyr¹¹⁷-Asn¹¹⁸-Thr¹¹⁹ of native KGF (as taught in commonly owned U.S.S.N. 08/323,473, filed on October 13, 1994). A still yet further example includes proteins having one or more amino acid substitutions, deletions or additions within a region of 123-133 (amino acids 154-164 of SEQ ID NO:2) of native KGF; these proteins may have agonistic or antagonistic activity.

Preferred muteins for practicing the present invention are selected from:
a) a polypeptide having residues corresponding to the cysteine at amino acid position 32 of SEQ ID NO:2 and the cysteine at amino acid position 46 of SEQ ID NO:2 replaced or deleted;
b) a charge-charge polypeptide wherein one or more of amino acid residues 72-185 of SEQ ID NO:2 are deleted or substituted with a neutral residue or negatively-charged residue selected to effect a protein with a reduced positive charge;
c) a polypeptide generated by substituting at least one amino acid having higher loop-forming potential for at least one amino acid within the loop-forming region of Asn¹⁴⁶-His¹⁴⁷-Tyr¹⁴⁸-Asn¹⁴⁹-Thr¹⁵⁰ of SEQ ID NO:2; or
d) a polypeptide having one or more amino acid substitutions, deletions or additions within amino acid residues 154-164 of SEQ ID NO:2.
Further preferred muteins are selected from, wherein "-" means a deletion in that position:
C(32,46)S, ΔN15, ΔN16, ΔN17, ΔN16, ΔN17, ΔN18, ΔN19, ΔN20, ΔN21, ΔN22, ΔN23, ΔN24, ΔN3/C(46)S, ΔN3/C(46)-, ΔN8/C(46)S, ΔN8/C(46)-, C(32,46)S/R(175)E, C(32,46)S/R(175)Q, ΔN23/R(175)Q, C(32,46,71)S, C(32,46,133)S, C(32,46,133,137)S, ΔN23/N(168)E, ΔN23/K(170)E, ΔN23/K(170)Q, ΔN23/R(175)A, ΔN23/R(175)E, ΔN23/R(175)L, ΔN23/K(178)E, ΔN23/K(178)Q, ΔN23(184)E, ΔN23(184)Q, ΔN23/Q(183)E/K(184)E, R(175)Q or H(147)G, with each mutein designated by reference to SEQ ID NO:2.

Even more preferred are KGF that include an initial methionine residue. ΔN23/K(147)E, ΔN23/K(147)Q, ΔN23/K(153)E, ΔN23/K(153)Q, ΔN23/Q(152)E/K(153)E; R(144)Q and H(116)G.

As those skilled in the art will also appreciate, a variety of host-vector systems may be utilized to express the KGF protein-coding sequence. These include but are not limited to eucaryotic cell systems such as mammalian cell systems infected with virus (*e.g*., vaccinia virus, adenovirus, etc.); insect cell systems infected with virus (*e.g.*, baculovirus); microorganisms such as yeast-containing yeast vectors; or to procaryotic cell systems such as bacteria transformed with bacteriophage DNA, plasmid DNA, or cosmid DNA. The expression elements of these vectors vary in their strengths and specificities. Depending on the host-vector system utilized, any one of a number of suitable transcription and translation elements may be used.

Once the protein product of KGF expression has been isolated, purified and assayed for KGF activity (using procedures known to those skilled in the art), it may be formulated in a variety of pharmaceutical compositions. Typically, such compositions include a suitable, usually chemically-defined, carrier or excipient for the therapeutic agent and, depending on the intended form of administration, other ingredients as well. The composition can include aqueous carriers or consist of solid phase formulations in which KGF is incorporated into non-aqueous carriers such as collagens, hyaluronic acid, and various polymers. The composition can be suitably formulated to be administered in a variety of ways, including by injection, orally, topically, intranasally and by pulmonary delivery.

### Brief Description of the Drawings

Figure 1 shows the nucleotide (SEQ ID NO:1) and amino acid (SEQ ID NO:2) sequences of native KGF (the nucleotides encoding the mature form of native KGF is depicted by bases 201 to 684 of SEQ ID NO:1 and the mature form of KGF is depicted by amino acid residues 32 to 194 of SEQ ID NO:2).
Figures 2A, 2B and 2C show the plasmid maps of pCFM1156, pCFM1656 and pCFM3102, respectively.
Figure 3 shows the nucleotide (SEQ ID NO:3) and amino acid (SEQ ID NO:4) sequences of the construct RSH-KGF.
Figure 4 shows the nucleotide (SEQ ID NO:5) and amino acid (SEQ ID NO:6) sequences of the construct contained in plasmid KGF.
Figure 5 shows the chemically synthesized OLIGOs (OLIGO#6 through OLIGO#11; SEQ ID NO:12-17, respectively) used to substitute the DNA sequence between a *KpnI* site and an *EcoRI* site for a *KpnI* site (from amino acid positions 46 to 85 of SEQ ID No:6) in the construct contained plasmid KGF to produce the construct in plasmid KGF(dsd).
Figure 6 shows the chemically synthesized OLIGOs (OLIGO#12 through OLIGO#24; SEQ ID NO:18-30, respectively) used to construct KGF(codon optimized).
Figure 7 shows the nucleotide (SEQ ID NO:31) and amino acid sequences (SEQ ID NO:32) of C(1,15)S, a KGF analog having substitutions of serine for cysteine at amino acid positions 1 and 15 of native KGF.
Figure 8 shows the nucleotide (SEQ ID NO:33) and amino acid sequences (SEQ ID NO:34) of R(144)Q, a KGF analog having a substitution of glutamic acid for arginine at amino acid position 144 of native KGF.
Figure 9 shows the nucleotide (SEQ ID NO:35) and amino acid (SEQ ID NO:36) sequences of C(1,15)S/R(144)Q, a KGF analog having substitutions of serine for cysteine at amino acid positions 1 and 15 and a substitution of glutamine for arginine at amino acid position 144 of native KGF.
Figure 10 shows the nucleotide (SEQ ID NO:37) and amino acid (SEQ ID NO:38) sequences of ΔN15, a KGF analog having a deletion of the first 15 amino acids of the N-terminus of native KGF.
Figure 11 shows the nucleotide (SEQ ID NO:39) and amino acid (SEQ ID NO:40) sequences of ΔN23, a KGF analog having a deletion of the first 23 amino acids of the N-terminus of native KGF.
Figure 12 shows the nucleotide (SEQ ID NO:41) and amino acid (SEQ ID NO:42) sequences of ΔN23/R(144)Q, a KGF analog having a deletion of the first 23 amino acids of the N-terminus and a substitution of glutamine for arginine at amino acid position 144 of native KGF.

### Description of Specific Embodiments

Standard methods for many of the procedures described in the following examples, or suitable alternative procedures, are provided in widely recognized manuals of molecular biology such as, for example, *Molecular Cloning,* Second Edition, Sambrook *et al.,* Cold Spring Harbor Laboratory Press (1987) and *Current Protocols in Molecular Biology,* Ausabel *et al.,* Greene Publishing Associates/Wiley-Interscience, New York (1990).

### Example 1: Preparation of DNA Coding for KGF and KGF Analogs

The cloning of the full-length human KGF gene (encoding a polypeptide with the sequence of native KGF) was carried out both by polymerase chain reaction (PCR) of RNA from an animal cell and by PCR of chemically synthesized (*E*. *coli* optimized codon) oligonucleotides ("OLIGOs"). Both procedures are described below:

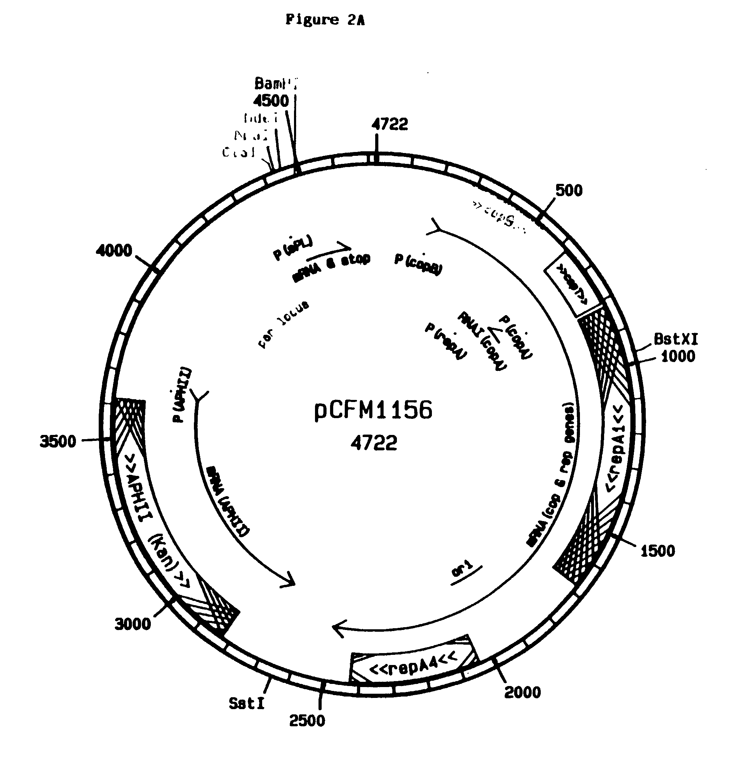

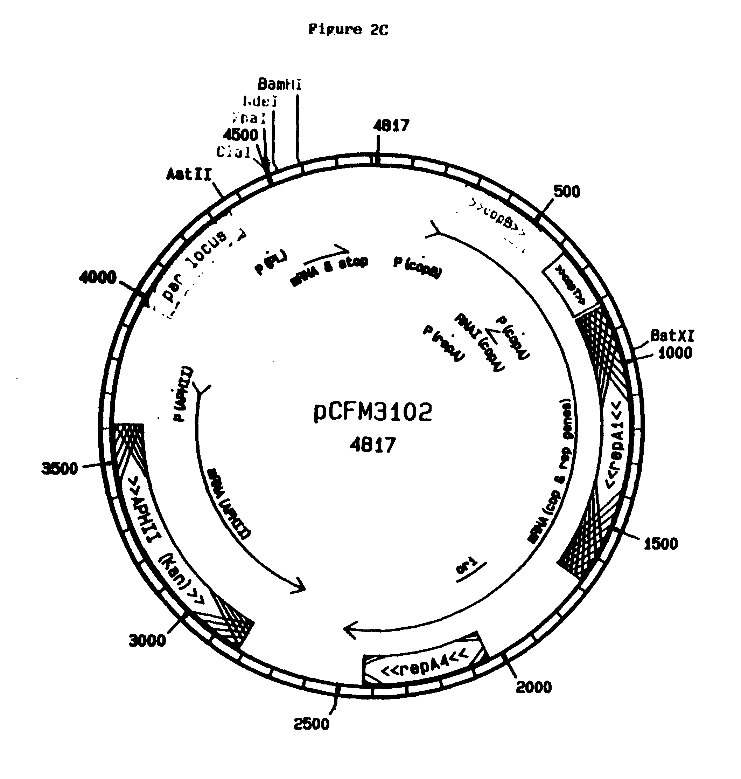

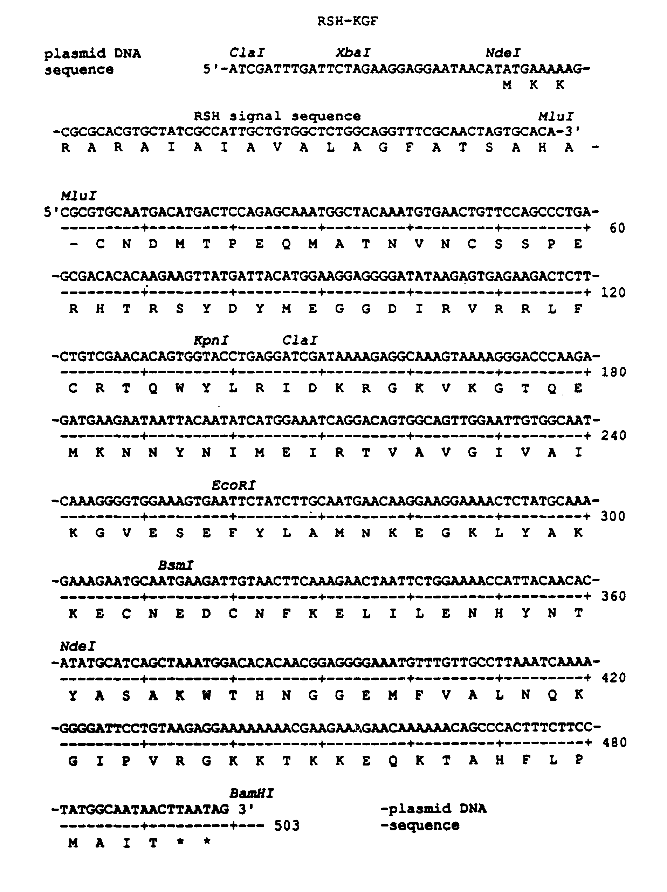

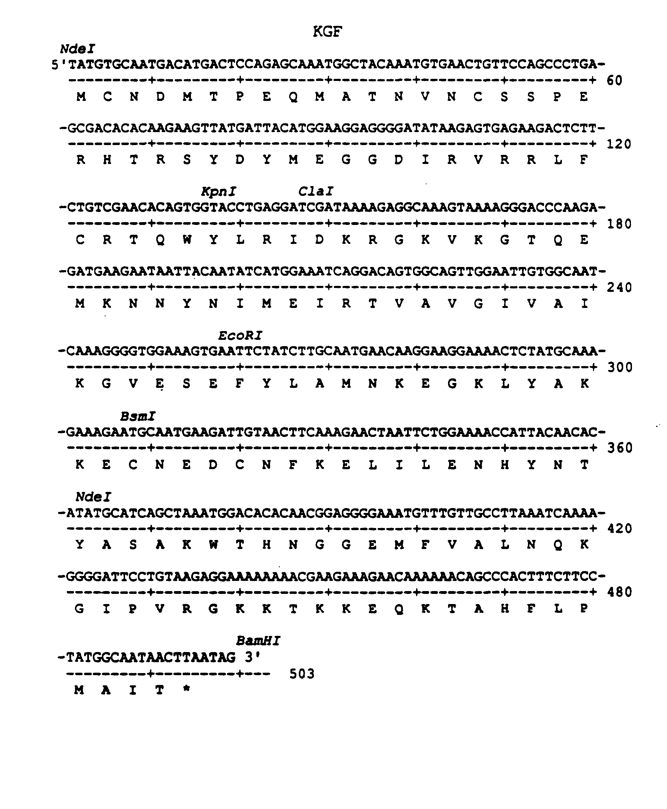

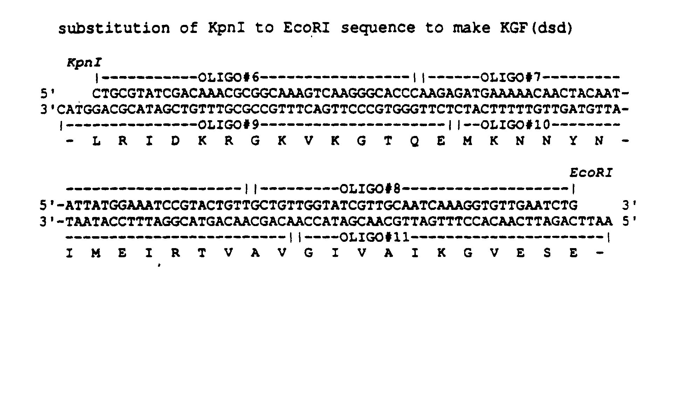

PCR amplification using RNA isolated from cells known to produce the polypeptide was performed. Initially, cells from a human fibroblast cell line AG1523A (obtained from Human Genetic Mutant Cell Culture Repository Institute For Medical Research, Camden, New Jersey) were disrupted with guanidium thiocyanate, followed by extraction (according to the method of Chomyzinski *et al.* (1987), *Anal. Biochem*., 172:156). Using a standard reverse transcriptase protocol for total RNA, the KGF cDNA was generated. PCR (PCR#1) amplification of the KGF gene was carried out using the KGF cDNA as template and primers OLIGO#1 and OLIGO#2 that encode DNA sequences immediately 5' and 3' of the KGF gene [model 9600 Thermocycler (Perkin-Elmer Cetus, Norwalk, CT); 28 cycles; each cycle consisting of one minute at 94°C for denaturation, two minutes at 60°C for annealing, and three minutes at 72°C for elongation]. A small aliquot of the PCR#1 product was then used as template for a second KGF PCR (PCR#2) amplification identical to the cycle conditions described above except for a 50°C annealing temperature. For expression cloning of the KGF gene, nested PCR primers were used to create convenient restriction sites at both ends of the KGF gene. OLIGO#3 and OLIGO#4 were used to modify the KGF DNA product from PCR#2 to include *MluI* and *BamHI* restriction sites at the 5' and 3' ends of the gene, respectively [PCR#3; 30 cycles; each cycle consisting of one minute at 94°C for denaturation, two minutes at 60°C for annealing, and three minutes at 72°C for elongation]. This DNA was subsequently cut with *MluI* and *BamHI,* phenol extracted and ethanol precipitated. It was then resuspended and ligated (using T4 ligase) into a pCFM1156 plasmid (Figure 2A) that contained a "RSH" signal sequence to make construct RSH-KGF (Figure 3). The ligation products were transformed (according to the method of Hanahan (1983), *J. Mol. Biol.,* 166:557) into *E*. *coli* strain FM5 (ATCC: 53911) and plated onto LB+kanamycin at 28°C. Several transformants were selected and grown in small liquid cultures containing 20 µg/mL kanamycin. The RSH-KGF plasmid was isolated from the cells of each culture and DNA sequenced. Because of an internal *NdeI* site in the KGF gene, it was not possible to directly clone the native gene sequence into the desired expression vector with the bracketed restriction sites of *NdeI* and *BamHI.* This was accomplished as a three-way ligation. Plasmid RSH-KGF was cut with the unique restriction sites of *BsmI* and *SstI*, and a ∼3 kbp DNA fragment (containing the 3' end of the KGF gene) was isolated following electrophoresis through a 1% agarose gel. A PCR (PCR#4) was carried out as described for PCR#3 except for the substitution of OLIGO#5 for OLIGO#3. The PCR DNA product was then cut with *NdeI* and *BsmI* and a 311 bp DNA fragment was isolated following electrophoresis through a 4% agarose gel. The third fragment used in the ligation was a 1.8 kbp DNA fragment of pCFM1156 cut with *NdeI* and *SstI* isolated following electrophoresis through a 1% agarose gel. Following ligation (T4 ligase), transformation, kanamycin selection and DNA sequencing as described above, a clone was picked containing the construct in Figure 4, and the plasmid designated KGF. Because of an internal ribosomal binding site that produced truncated products, the KGF DNA sequence between the unique *KpnI* and *EcoRI* sites was replaced with chemically synthesized OLIGOs (OLIGO#6 through OLIGO#11) to minimize the use of the internal start site (Figure 5).

The OLIGOs were phosphorylated with T4 polynucleotide kinase and then heat denatured. The single-stranded (ss) OLIGOs were then allowed to form a ds DNA fragment by allowing the temperature to slowly decrease to room temperature. T4 ligase was then used to covalently link both the internal OLIGO sticky-ends and the whole ds OLIGO fragment to the KGF plasmid cut with *KpnI* and *EcoRI*. The new plasmid was designated KGF(dsd).

A completely *E. coli* codon-optimized KGF gene was constructed by PCR amplification of chemically synthesized OLIGOs #12 through 24.

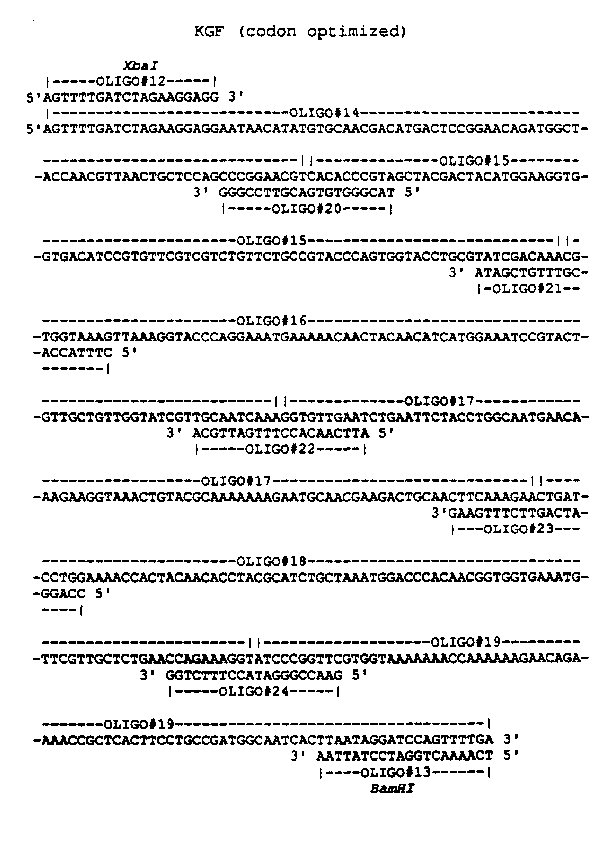

OLIGOs #12 through 24 were designed so that the entire DNA sequence encoding native KGF was represented by OLIGOs from either the "Watson" or the "Crick" strand and upon PCR amplification would produce the desired double-stranded DNA sequence (Figure 6) [PCR#5, Model 9600 thermocycler (Perkin-Elmer Cetus); 21 cycles, each cycle consisting of 31 seconds at 94°C for denaturation, 31 seconds at 50°C for annealing, and 31 seconds at 73°C for elongation; following the 21 cycles the PCR was finished with a final elongation step of 7 minutes]. After PCR amplification, the DNA fragment was cut with *XbaI* and *BamHI* and the 521 bp fragment ligated into the expression plasmid pCFM1156 cut with the same enzymes. PCR#5 utilized the outside primers (100 pmoles/100 µl rxn) OLIGO#12 and OLIGO#13 and 1 µl/100 µl rxn of a KGF template derived by ligation (by T4 ligase) of OLIGOs #14 through #19 (OLIGOs#15 through OLIGOs#18 were phosphorylated with T4 polynucleotide kinase) using OLIGOs#20 through OLIGOs#24 as band-aid oligos (Jayaraman *et al.* (1992), *Biotechniques,* 12:392) for the ligation. The final construct was designated KGF(codon optimized).

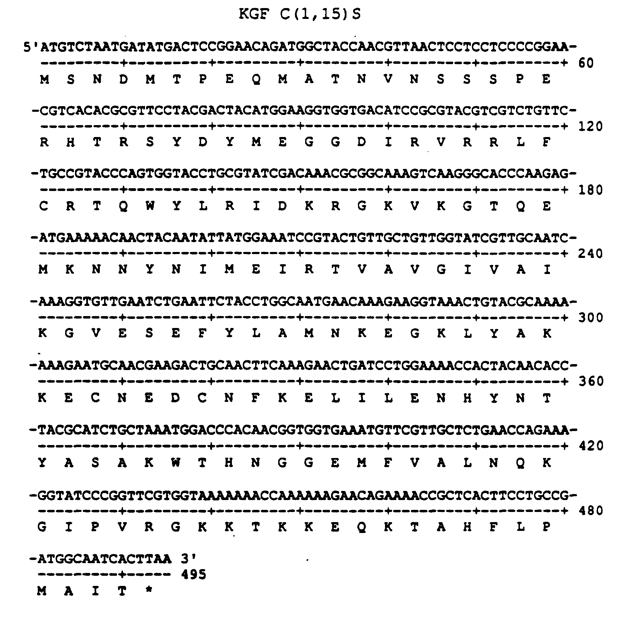

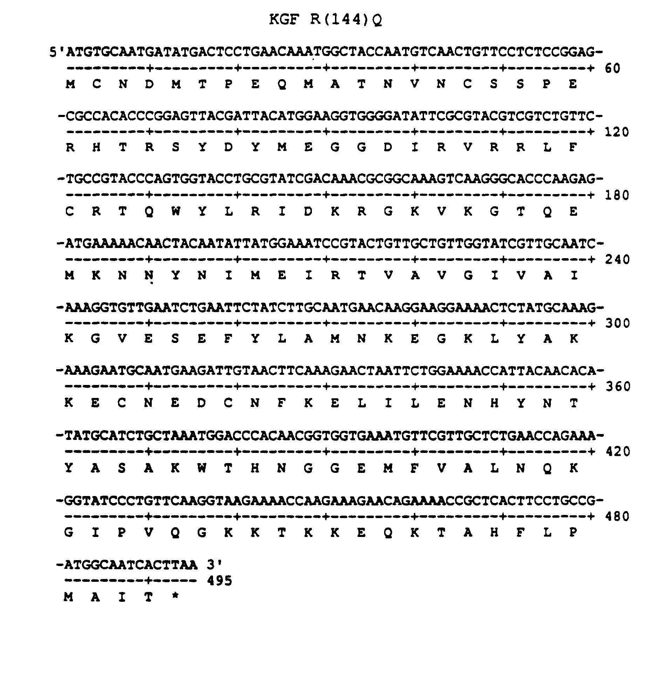

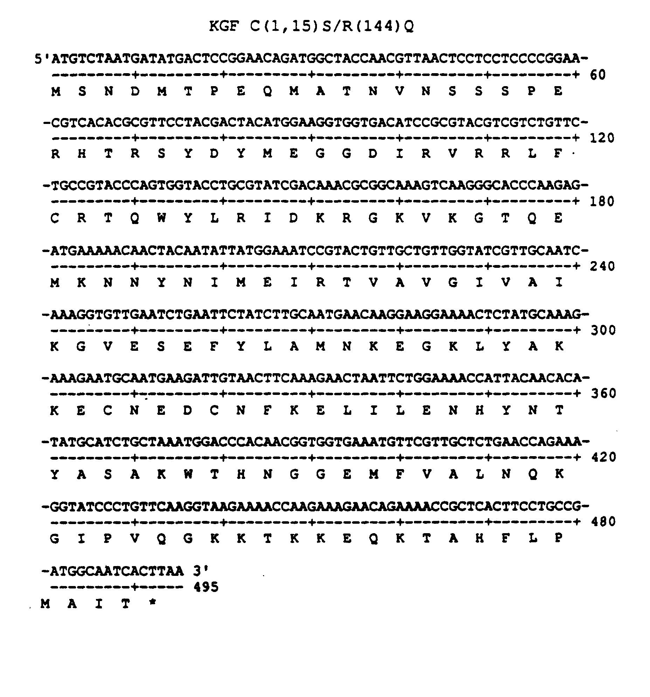

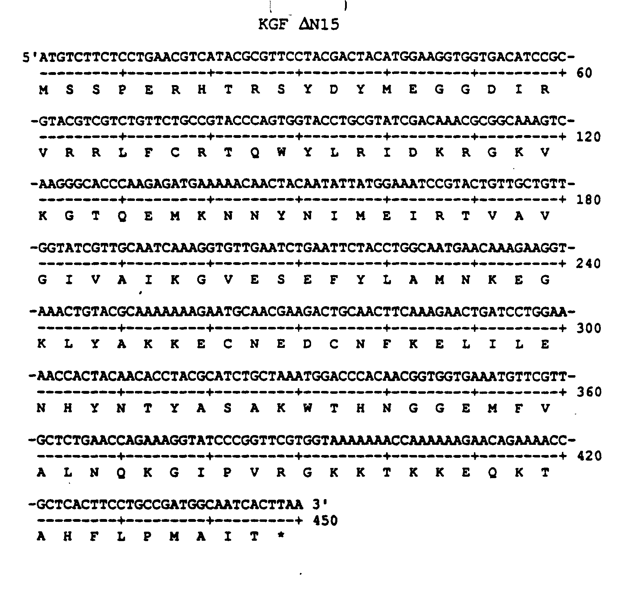

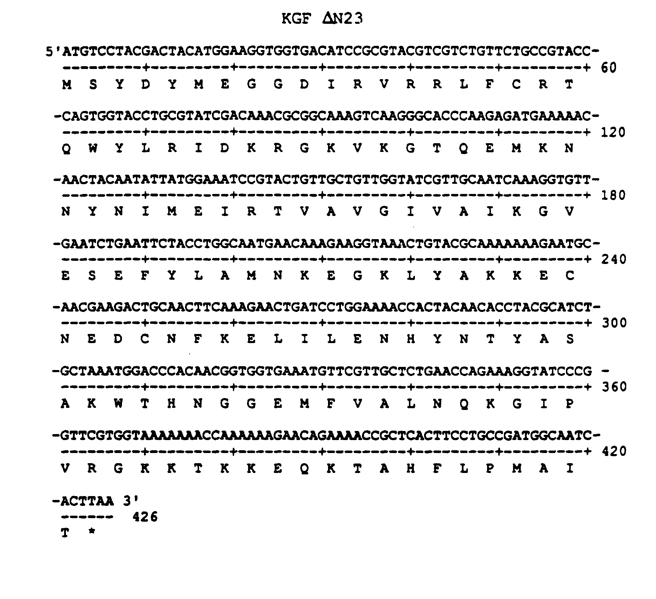

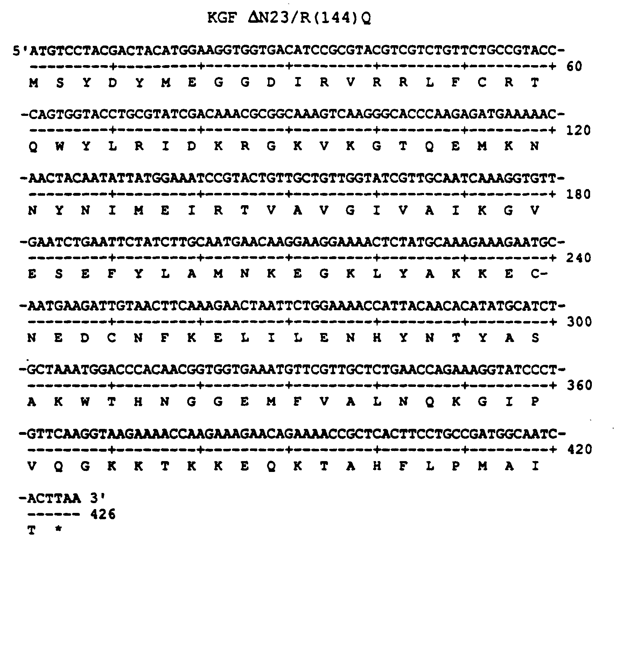

All of the KGF analogs described herein are composed in part from DNA sequences found in KGF(dsd) or KGF(codon optimized), or a combination of the two. The sequences are further modified by the insertion into convenient restrictions sites of DNA sequences that encode the particular KGF analog amino acids made utilizing one or more of the above-described techniques for DNA fragment synthesis. Any of the analogs can be generated in their entirety by either of the above described techniques. However, as a part of the general OLIGO design optimized *E. coli* codons were used where appropriate, although the presence of *E. coli* optimized codons in part or *in toto* of any of the genes where examined did not significantly increase the yield of protein that could be obtained from cultured bacterial cells. Figures 7 to 12 set forth by convenient example particular KGF analog nucleotide and amino acid sequence constructions: C(1,15)S (Figure 7) ; R(144)Q (Figure 8) ; C(1,15)S/R(144)Q (Figure 9); ΔN15 (Figure 10); ΔN23 (Figure 11) and ΔN23/R(144)Q (Figure 12). All the KGF analog constructions described herein were DNA sequence confirmed.

### Example 2: Purification from E. coli

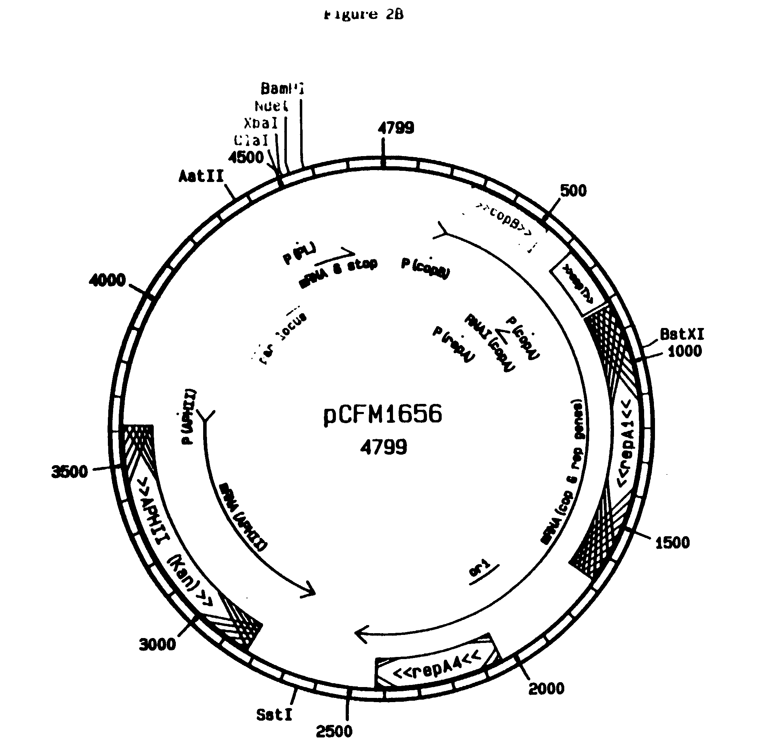

Three different expression plasmids were utilized in the cloning of the KGF analog genes. They were pCFM1156 (ATCC 69702), pCFM1656 (ATCC 69576), and pCFM3102 (Figures 2A, 2B and 2C, respectively). The plasmid p3102 can be derived from the plasmid pCFM1656 by making a series of site directed base changes with PCR overlapping oligo mutagenesis. Starting with the *BglII* site (pCFM1656 plasmid bp #180) immediately 5' to the plasmid replication promoter, P_{copB,} and proceeding toward the plasmid replication genes, the base pair changes are as follows:

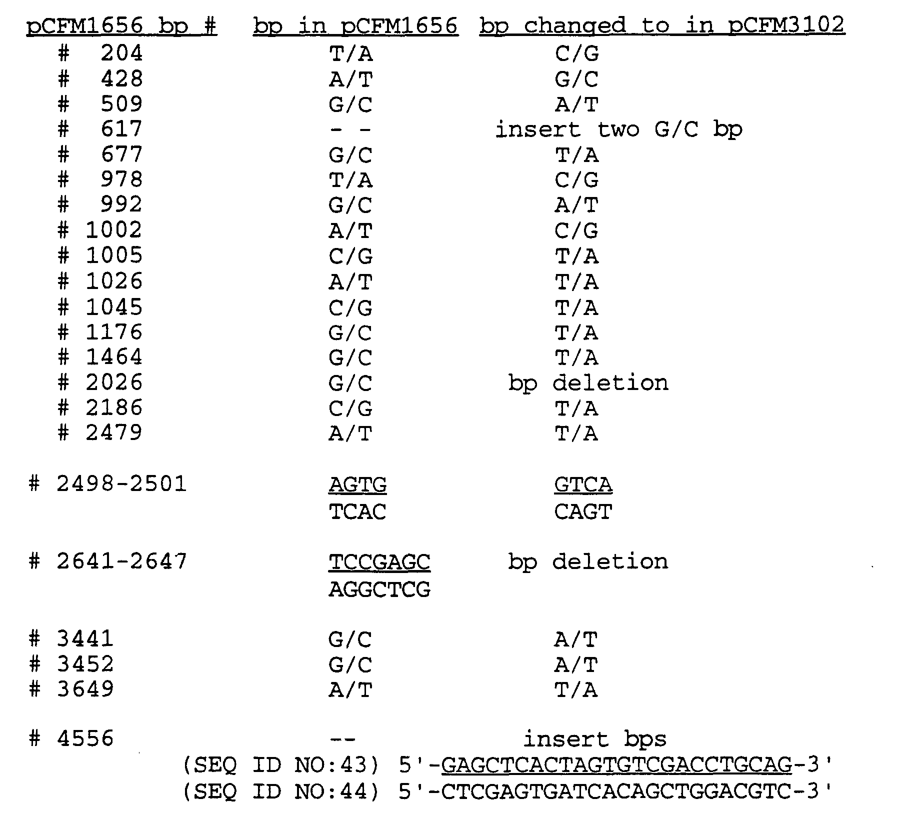

As seen above, pCFM1156, pCFM1656 and pCFM3102 are very similar to each other and contain many of the same restriction sites. The plasmids were chosen by convenience, and the vector DNA components can be easily exchanged for purposes of new constructs. The host used for all cloning was *E*. *coli* strain FM5 (ATCC: 53911) and the transformations were carried out (according to the method of Hanahan (1983), *supra)* or by electroelution with a Gene Pulser™ transfection apparatus (BioRad Laboraties, Inc., Hercules, CA), according to the manufacturer's protocol.

Initially, a small, freshly-cultured inoculum of the desired recombinant *E*. *coli* clone harboring the desired construct on one of the three pCFM vectors was started by transferring 0.1 mL of a frozen glycerol stock of the appropriate strain into a 2 L flask containing 500 mL of Luria broth. The culture was shaken at 30°C for 16 hours. Thereafter the culture was transferred to a 15 L fermentor containing 8 L of sterile batch medium (Tsai, *et al*. (1987), *J. Industrial Microbiol.,* 2:181-187).

Feed batch fermentation starts with the feeding of Feed # 1 medium (Tsai, *et al.* (1987.), *supra).* When the OD600 reached 35, expression of the desired KGF analog was induced by rapidly raising the culture temperature to 37°C to allow the amplification of plasmid. After two hours at 37°C, the culture temperature was quickly raised to 42°C to denature the CI repressor and the addition of Feed 1 was discontinued in favor of Feed 2, the addition rate of which was initiated at 300 mL/hr. Feed 2 comprised 175 g/L trypticase-peptone, 87.5 g/L yeast extract, and 260 g/L glucose. After one hour at 42°C, the culture temperature was decreased to 36°C, where this temperature was then maintained for another 6 hours.

The fermentation was then halted and the cells were harvested by centrifugation into plastic bags placed within 1 L centrifuge bottles. The cells were pelleted by centrifugation at 400 rpm for 60 minutes, after which the supernatants were removed and the cell paste frozen at -90°C.

Following expression of the various KGF analogs in *E*. *coli,* native KGF, C(1,15)S, R(144)Q, C(1,15)S/R(144)Q, ΔN15, ΔN23, and ΔN23/R(144)Q protein were purified using the following procedure. Cell paste from a high cell density fermentation was suspended at 4°C in 0.2 M NaCl, 20 mM NaPO₄, pH 7.5 as a 10-20% solution (weight per volume) using a suitable high shear mixer. The suspended cells were then lysed by passing the solution through a homogenizer (APV Gaulin, Inc., Everett, MA) three times. The outflowing homogenate was cooled to 4-8°C by using a suitable heat exchanger. Debris was then removed by centrifuging the lysate in a J-6B™ centrifuge (Beckman Instruments, Inc., Brea, CA) equipped with a JS 4.2 rotor at 4,200 rpm for 30-60 min. at 4°C. Supernatants were then carefully decanted and loaded onto a previously prepared 450 mL (5 cm x 23 cm) column of S-Sepharose Fast Flow™ resin (Pharmacia) column equilibrated with 0.2 M NaCl, 20 mM NaPO₄, pH 7.5 at 4°C. Next, the column was washed with five column volumes (2250 mL) of 0.4 M NaCl, 20 mM NaPO₄, pH 7.5 at 4°C. The desired protein was eluted by washing the column with 5 L of 0.5 M NaCl, 20 mM NaPO₄, pH 7.5. Again, 50 mL fractions were collected and the A₂₈₀ of the effluent was continuously monitored. Fractions identified by A₂₈₀ as containing eluted material were then analyzed by SDS-PAGE through 14% gels to confirm the presence of the desired polypeptide.

Those fractions containing proteins of interest were then pooled, followed by the addition of an equal volume of distilled water. The diluted sample was then loaded onto a previously prepared 450 mL (5 cm x 23 cm) column of S-Sepharose Fast Flow equilibrated with 0.4 M NaCl, 20 mM NaPO₄, pH 6.8 at 4°C. The column was washed with 2250 mL of 0.4 M NaCl, 20 mM NaPO₄, pH 6.8 and the protein eluted using a 20 column volume linear gradient ranging from 0.4 M NaCl, 20 mM NaPO₄, pH 6.8 to 0.6 M NaCl, 20 mM NaPO₄, pH 6.8. Again, 50 mL fractions were collected under constant A₂₈₀ monitoring of the effluent. Those fractions containing the protein (determined by 14% SDS-PAGE) were then pooled, followed by concentration through a YM-10 membrane (10,000 molecular weight cutoff) in a 350cc stirring cell (Amicon, Inc. Mayberry, MA) to a volume of 30-40 mL.

The concentrate was then loaded onto a previously generated 1,300 mL (4.4 cm x 85 cm) column of Superdex-75™ resin (Pharmacia) equilibrated in column buffer comprising 1X PBS (Dulbecco's Phosphate Buffered Saline, "D-PBS," calcium and magnesium-free) or 0.15 M NaCl, 20 mM NaPO₄, pH 7.0. After allowing the sample to run into the column, the protein was eluted from the gel filtration matrix using column buffer. Thereafter, 10 mL fractions were recovered and those containing the analog (determined by 14% SDS-PAGE) were pooled. Typically, the protein concentration was about 5-10 mg/mL in the resultant pool. All of the above procedures were performed at 4-8°C, unless otherwise specified.

An alternative purification procedure was used to purify native KGF, C(1,15)S and ΔN23. The procedure involves the following steps and, unless otherwise specified, all procedures, solutions and materials were conducted at 23 ± 5°C.

Upon completion of the production phase of a bacterial fermentation, the cell culture was cooled to 4-8°C and the cells were harvested by centrifugation or a similar process. On the basis of the expected yield of protein per unit weight of cell paste and the amount of purified protein required, an appropriate amount of cell paste, by weight, was suspended in a mild buffer solution, 20 mM NaPO₄, 0.2 M NaCl, pH 7.5, weighing about five times that of the cell paste to be suspended. The cells were dispersed to a homogeneous solution using a high shear mixer. The temperature of the cell paste dispersion was maintained at 4-8°C during homogenization.

The cells were then lysed by pressure, for example by passing the cell paste dispersion twice through an appropriately sized cell homogenizer. The homogenate was kept chilled at 5 ± 3°C. To clarify the cell lysate, a previously prepared depth filter housing (Cuno, Inc., Meriden, CT) equipped with a filter having an appropriate amount of filter surface area, equilibrated with a suitable volume of 0.2 M NaCl, 20 mM NaPO₄, pH 7.5 was employed. The equilibration and clarification were performed at 5 ± 3°C. Prior to clarification, an appropriate amount of a suitable filter aid was used to pre-coat the filter and be thoroughly mixed with the cell lysate, after which the lysate was clarified by passing the solution through the filter apparatus. The filter was washed with 0.2 M NaCl, 20 mM NaPO₄, pH 7.5. The filtrate and any subsequent wash were collected in a chilled container of suitable capacity, all the while being maintained at less than 10°C.

Following clarification the lysate was then passed through a previously prepared column of SP-Sepharose Fast Flow containing at least 1 mL of resin per 2 g of cell paste. The column of SP-Sepharose Fast Flow was equilibrated with cold (5 ± 3°C), 0.2 M NaCl, 20 mM NaPO₄, pH 7.5. The temperature of the column was maintained at less than 10°C. The clarified lysate (5 ± 3°C) was then loaded onto the ion exchange column, with the absorbance at 280 nm (A₂₈₀) of eluate being continuously monitored. After sample loading, the column was washed with cold 0.2 M NaCl, 20 mM NaPO₄, pH 7.5, followed by washing with 0.3 M NaCl, 20 mM NaPO₄, pH 7.5 at 23 ± 5°C.

To elute the desired protein, a linear gradient ranging from 0.2-1 M NaCl, 20 mM NaPO₄, pH 7.5 was used. Bulk product was collected in several fractions on the basis of the A₂₈₀ of the eluate. Following elution, these fractions were pooled and the volume noted.

To oxidize free sulfhydryl groups, an oxidation step was performed. For proteins with altered cysteine patterns, as compared to native KGF, an oxidizing agent (e.g., cystamine dihydrochloride or another appropriate oxidizing agent, for instance, cystine, oxidized glutathione or divalent copper) was added to a final concentration of 1-20 mM and the pH was adjusted to 7-9.5, with a pH of 9.0 ± 0.3 when cystamine dihydrochloride was used. The oxidation was conducted at 10 - 30'C for an appropriate period. For the native KGF protein, oxidation was accomplished by adding an appropriate amount of (NH₄)₂SO₄ such as 1-2 M (NH₄)₂SO₄, adjusting the pH to 7.5-9.5, and holding the temperature at 23 ± 5°C for an appropriate period.

After oxidation, the pH of the solution was adjusted to between 6.5 and 9.5. If necessary, solid (NH₄)₂SO₄ was added to the solution to a final concentration of 2 M. To remove particulates, the solution was passed through appropriate clarification filters.

The filtered, oxidized product was then subjected to hydrophobic interaction chromatography (HIC). The HIC matrice was Butyl-650M Toyopearl™ resin (Tosohaas, Inc., Montgomeryville, PA). The protein-containing solution was loaded onto the column, which had been previously equilibrated with 2 M (NH₄)₂SO₄, 0.15 M NaCl, 20 mM NaPO₄, pH 7.0. After sample loading, the column was washed with 2 M (NH₄)₂SO₄, 0.15 M NaCl, 20 mM NaPO₄, pH 7.0. The desired protein was then eluted using a decreasing linear (NH₄)₂SO₄ gradient ranging from 2-0 M developed in 0.15 M NaCl, 20 mM NaPO4, pH 7.0. When the desired protein began to elute, as indicated by an increase in the A₂₈₀ of the eluate, fractions were collected. Aliquots of each fraction were then analyzed by SDS-PAGE. Those fractions containing the desired protein were then pooled, thoroughly mixed, and the volume of the pool determined, as was the concentration of the protein therein.

The pooled HIC protein-containing eluate was then concentrated and the elution buffer exchanged. Typically, proteins were concentrated to 5.0-10.0 mg/mL. Ultrafiltration was conducted using an ultrafiltration system equipped with a Pellicon™ cassette system (Millipore, Inc., Bedford, MA) with an appropriately sized cut-off membrane

After concentration, the sample was diafiltered against an appropriate buffer. The retentate from the concentration step was diafiltered against 0.15 M NaCl, 20 mM NaPO₄, pH 7.0 until the conductivity of the retentate was within 5% of the conductivity of the 0.15 M NaCl, 20 mM NaPO₄, pH 7.0 solution.

In addition, to remove precipitates and bacterial endotoxin that might be present, the concentrated diafiltered protein-containing sample was passed through a 0.1 µm Posidyne™ filter (Pall, Inc., Cortland, NY). After determining the protein concentration of the solution and on the basis of the desired concentration of the final bulk product, the solution was diluted with 0.15 M NaCl, 20 mM sodium phosphate, pH 7.0, to the desired final concentration. A final aseptic filtration through a 0.22 µm filter, was then performed as the final bulk product was transferred to a pyrogen-free container for storage (at about 5°C) for further formulation.

### Example 3: Purification from Mammalian Cell Culture

This example describes the expression, isolation, and characterization of two biologically active recombinant KGF (rKGF) forms produced in a mammalian expression system.

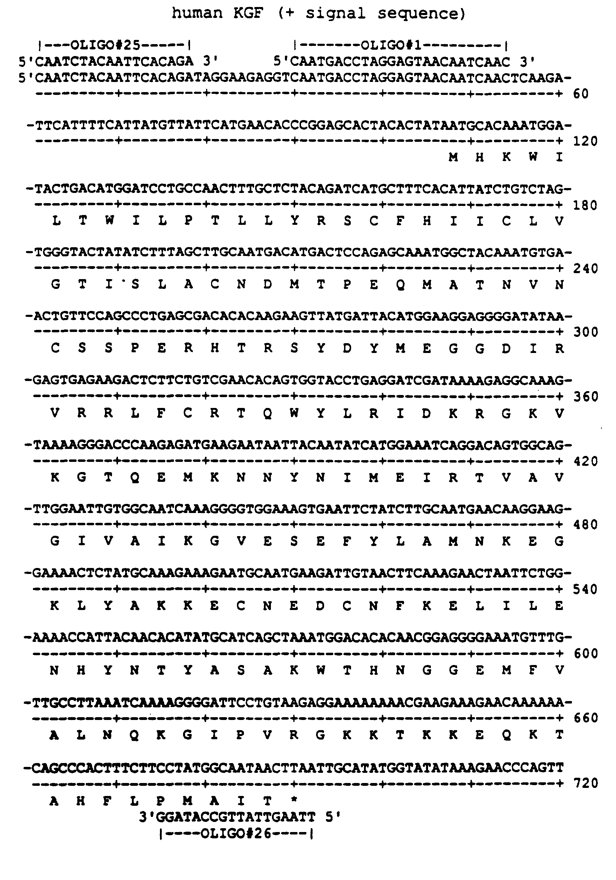

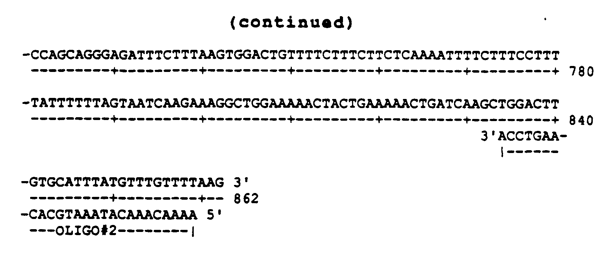

The human KGF gene was isolated by PCR amplification of cDNA made from normal dermal human fibroblast cells (Clonetec, Inc., Palo Alto, CA). Following the making of cDNA by reverse transcriptase, PCR was used to amplify the KGF gene. OLIGO#25 and OLIGO#26 were used to amplify the gene out of the cDNA and OLIGO#27 and OLIGO#28 were used to place *Hind*III and *Bgl*II restriction sites at the fragment ends by a second PCR amplification, as set forth in Figure 1.

Following cloning and DNA sequence confirmation, the KGF gene DNA was then used. Amplification was effected using two primers:

The sense primer, OLIGO#29, included an *Xba*I site and a consensus Kozak translation sequence (5'-CCACC-3') upstream of the start codon, ATG. The antisense primer, OLIGO#30, included a *Sal*I cloning site and an additional stop codon. After 18 cycles of PCR amplification (30 sec. denaturation at 94°C, 40 sec. annealing at 55°C, and 40 sec. elongation at 72°C), the product was digested with *Xba*I and *Sal*I and ligated with a similarly digested DNA of pDSRα2 (according to the methods of Bourdrel *et al*. (1993), *Protein Exp*. *& Purif.,* 4:130-140 and Lu *et al*. (1992), *Arch. Biochem. Biophys*., 298:150-158). This resulted in plasmid KGF/pDSRα2 which placed the human KGF gene between the SV40 early promoter and the α-FSH polyadenylation sequences. Two clones were picked and DNA sequence analysis confirmed construction of the desired vector.

Two micrograms of KGF/pDSRα2 DNA were then linearized with *Pvu*I. Chinese hamster ovary (CHO) cells, seeded the day before at 0.8 x 10⁶ cells/60 mm culture dish, were then transfected with the treated DNA using a standard calcium phosphate precipitation method (Bourdrel *et al*., *supra*). Two weeks later, individual colonies were picked and transferred into 24-well plates. The conditioned media was considered serum-free when the cells reached confluency and aliquots thereof were analyzed by Western blotting using a polyclonal rabbit antiserum reactive against *E. coli*-expressed human KGF.

Westerns were performed by running samples through 12.5% (w/v) SDS polyacrylamide gels, followed by electroblotting for 1 hr. at 400 mA onto nitrocellulose membranes using a semidry transfer apparatus (Hoefer Scientific Instruments, San Francisco, CA). 20 mM Tris, 150 mM glycine, 20% methanol served as the transfer buffer. The nitrocellulose sheets were blocked by incubation with 10% normal goat serum in PBS. Rabbit anti-serum raised against *E. coli*-derived KGF was used as primary antibody. For use, it was diluted 1/10,000 in 1% normal goat serum in PBS and incubated with the blocked nitrocellulose sheets for 12 hr. at room temperature, after which excess antibody was removed by three 30 min. washes in PBS. The nitrocellulose membranes were then incubated in 100 mL of 1% normal goat serum in PBS containing Vectastain™ biotinylated goat anti-rabbit IgG (secondary antibody, Vector Labs, Burlingame, CA), for 30 minutes at room temperature. After three 10 minute washes in PBS, a 30 minute room temperature incubation was performed in a 100 mL solution of 1% normal goat serum containing streptavidin and biotinylated peroxidase, prepared according to manufacturer's directions (Vector Labs). Following three washes in PBS, KGF cross-reactive material was visualized by incubation in a mixture of 60 µL of 30% (w/v) H₂O₂ in 100 mL of PBS and 50 mg of 4-chloronapthol in 20 mL of methanol. The reaction was stopped by rinsing in water after 10 minutes.

Analysis of the blots revealed that the KGF-specific antibody associated with three distinct protein bands, two being closely related with molecular weights of about 25-29 kDa and one with an estimated molecular weight of about 17 kDa, as compared to the expected molecular weight of approximately 18.8 of the 163 amino acid mature protein. Additionally, several high expressing clones secreting more than 2.0 mg of rKGF per liter, as judged by Western analysis, were selected and expanded into roller bottles (according to the method of Lu *et al*., *supra)* to generate large volumes of serum-free conditioned medium for purification of KGF by cationic exchange chromatography and gel filtration, as set forth below.

KGF from 3 L of serum-free conditioned medium was purified applying the medium directly to a cation exchange column (5 x 24 cm) packed with 450 mL of sulfoethyl column of SP-Sepharose Fast Flow(Pharmacia) pre-equilibrated with 20 mM sodium phosphate, pH 7.5. After washing with five column volumes of 20 mM sodium phosphate, 0.2 M NaCl, pH 7.5, rKGF was eluted using a 20 column volume linear gradient of 0.2 to 1.0 M NaCl in 20 mM sodium phosphate, pH 7.5. 50 mL fractions were collected with continuous A₂₈₀ monitoring. KGF protein was detected by analyzing aliquots of each fraction by SDS-PAGE. SDS-PAGE was performed on an electrophoresis system (Novex, San Diego, CA) using precast 14% Tris-glycine precast gels (according to the method of Laemmli (1970), *Nature,* 227:680-685). Samples were mixed with non-reducing SDS sample buffer without heating before loading. The proteins were detected by either Coomassie blue or silver staining. Two late-eluting peaks were seen to contain protein bands corresponding to the 25-29 kDa and 17 kDa bands detected by Western blot. The fractions containing each of these peaks were separately concentrated to a volume of less than 1.0 mL and subjected to gel filtration.

The gel filtrations employed columns of Superdex-75™ resin (HR 10/30, Pharmacia) pre-equilibrated with PBS, pH 7.2, and calibrated with the following known molecular weight standards (BioRad, San Francisco, CA): thyroglobulin (670 kDa), gamma globulin (158 kDa), ovalbumin (44 kDa), myoglobin (17 kDa) and vitamin B-12 (1.4 kDa). These purification steps resulted in an approximate 2000-fold purification of rKGF, specifically including a 17 kDa and a 30 kDa material, as estimated by silver staining.

In the instance of the higher molecular weight material, rKGF eluted as a major symmetrical peak, which was called KGF-a. Upon SDS-PAGE analysis of a lesser amount of this material, 3 µg/lane versus 6 µg/lane, two bands with a 1-2 kDa molecular weight difference were resolved. In the instance of the lower molecular weight material, termed KGF-b, gel filtration resulted in a protein preparation having the expected mobility. For both KGF-a and KGF-b, the overall yield after purification was approximately 30-40%.

Amino acid sequences from KGF-a and KGF-b were also analyzed. These analyses were performed on an automatic sequencer (Model 477A or 470A, Applied Biosystems, Inc., Foster City, CA) equipped with a Model 120A on-line PTH-amino acid analyzer and a Model 900A data collection system (according to the method of Lu *et al.* (1991), *J. Biol. Chem.,* 266:8102-8107). Edman sequence analysis of KGF-a revealed a major N-terminal sequence of X₁-N-D-M-T-P-E-Q-M-A-T-N-V-X₂-X₃-S- (SEQ ID NO:51). A minor sequence starting from the third N-terminal amino acid, aspartic acid, was also present in 1.6% of the total sequenceable protein. X₁, X₂, and X₃ were the unassigned due to the absence of phenylthiohydantoinyl (PTH) amino acid signals during sequence analysis.

Interestingly, N-terminal sequence analysis of KGF-b revealed an N-terminal amino acid sequence of S-Y-D-Y-M-E-G-G-D-I-R-V- (SEQ ID NO:52), indicating that it is an N-terminally truncated form of KGF that has been proteolytically cleaved at the Arg²³-Ser²⁴ peptide bond.

To further characterize purified KGF-a and KGF-b, the protein was subjected to glycosidases (neuraminidase, O-glycanase, and/or N-glycanase), using known techniques (Sasaki *et al.* (1987), *J. Biol. Chem.,* 262:12059-12076; Takeuchi *et al.* (1988), *J. Biol. Chem*., 263:3657-3663; Zsebo *et al.* (1990), *Cell,* 63:195-201). These data indicate that KGF-a contains N- and O-linked carbohydrates, although the lower molecular weight form of KGF-a probably contains only N-linked sugar. Glycosidase treatment did not cause molecular weight reduction for KGF-b, indicating that the molecule is unglycosylated.

### Example 4: Biological Activity

Each KGF analog was diluted and assayed for biological activity by measuring the [³H]-thymidine uptake of Balb/MK cells (according to the method of Rubin *et al.* (1989), *supra).* The samples were first diluted in a bioassay medium consisting of 50% customer-made Eagle's MEM, 50% customer-made F12, 5 µg/mL transferrin, 5 ng/mL sodium selenite, 0.0005% HSA and 0.005% Tween 20. KGF samples were then added into Falcon Primeria 96-well plates seeded with Balb/MK cells. Incorporation of [³H]-Thymidine during DNA synthesis was measured and converted to input native KGF concentration by comparison to a native KGF standard curve. Each of the tested analogs exhibited mitogenic activity.

Interaction with the KGF receptor was examined using isolated KGF receptor membrane preparations prepared from Balb/MK mouse epidermal keratinocytes (by the procedure described by Massague (1983), *J. Biol. Chem*., 258:13614-13620). Specifically, various forms of KGF were diluted with 50 mM Tris-HCl, pH 7.5, containing 0.2% bovine serum albumin so as to range in concentration from 0.8 ng to 100 ng per 50 µL. They were individually incubated with the membrane preparation (75 ng/mL) and ¹²⁵I-labeled *E*. *coli*-derived KGF (1.5 ng). Receptor binding and competition experiments were performed at 4°C for 16 hr., after which time samples were taken, centrifuged, and washed twice with the above diluent buffer to remove unbound and non-specifically bound, labeled KGF. Samples were then counted for the remaining radioactivity. Competition curves for receptor binding between KGF samples and labeled KGF were constructed by plotting percent uncompetition versus concentrations of each KGF sample. Radioreceptor assay uncompetition experiments indicated that *E*. *coli*-derived KGF, KGF-a, and KGF-b have similar receptor binding activity.

## Claims

1. A method for purifying a keratinocyte growth factor (KGF), the method comprising the following steps:
a) obtaining a solution comprising the KGF;
b) loading the solution of step a) onto a cation exchange resin;
c) eluting the KGF in an eluate solution from the cation exchange resin;
d) passing the eluate solution from step c) through either (i) a molecular weight exclusion matrix, or (ii) a hydrophobic interaction chromatography matrix; and
e) recovering the KGF,
with the proviso that said method does not comprise a heparin affinity chromatography step.

2. The method according to claim 1, wherein the cation exchange resin is selected from a carboxymethyl cellulose, carboxymethyl agarose, sulfated agarose or cellulose column.

3. The method according to any one of claims 1 to 2, wherein the eluate solution from step c) is passed through a molecular weight exclusion matrix.

4. The method according to claim 3, wherein the molecular weight exclusion matrix is selected from agarose-based, acrylamide-based, silica-based or polymer-based size-exclusion columns.

5. The method according to any one of claims 1 or 2, wherein the eluate solution from step c) is passed through a hydrophobic interaction chromatography matrix.

6. The method according to claim 5, wherein free sulfhydryl groups of the KGF in the eluate solution are oxidized prior to passing the eluate solution through the hydrophobic interaction chromatography matrix.

7. The method according to claim 6, wherein the KGF is oxidized by being contacted with an oxidizing agent.

8. The method according to claim 7, wherein the oxidizing agent is selected from cystamine dihydrochloride, cystine, oxidized glutathione or divalent copper.

9. The method according to any one of claims 1, 2, and 5 to 8, wherein the hydrophobic interaction chromatography matrix is selected from a column having an alkyl- or phenyl-substituted resin.

10. The method according to any one of claims 1 to 9, wherein said KGF comprises amino acid residues 32-194 of SEQ ID NO:2, optionally with a signal sequence, or a mutein thereof.

11. The method of claim 10, wherein said mutein of keratinocyte growth factor is selected from
a) a polypeptide having residues corresponding to the cysteine at amino acid position 32 of SEQ ID NO:2 and the cysteine at amino acid position 46 of SEQ ID NO:2 replaced or deleted;
b) a charge-change polypeptide wherein one or more of amino acid residues 72-185 of SEQ ID:2 are deleted or substituted with a neutral residue or negatively-charged residue selected to effect a protein with a reduced positive charge;
c) a polypeptide generated by substituting at least one amino acid having higher loop-forming potential for at least one amino acid within the loop-forming region of Asn¹⁴⁶-His¹⁴⁷-Tyr-¹⁴⁸-Asn-¹⁴⁹-Thr¹⁵⁰ of SEQ ID NO:2; or
d) a polypeptide having one or more amino acid substitutions, deletions or additions within amino acid residues 154-164 of SEQ ID NO:2.

12. The method of claim 11, wherein said mutein is selected from C(32,46)S, ΔN15, ΔN16, ΔN17, ΔN18, ΔN19, ΔN20, ΔN21, ΔN22, ΔN23, ΔN 24, ΔN3/C(46)S, ΔN3/C(46)-, ΔN8/C(46)S, ΔN8/C(46)-, C(32,46)S/R(175)E, C(32,46)S/R(175)Q, ΔN23/R(175)Q, C(32,46,71)S, C(32,46,133)S, C(32,46,133,137)S, ΔN23/N(168)E, ΔN23/K(170)E, ΔN23/K(170)Q, ΔN23/R(175)A, ΔN23/R(175)E, ΔN23/R(175)L, ΔN23/K(178)E, ΔN23/K(178)Q, ΔN23(184)E, ΔN23/K(184)Q, ΔN23/Q(183)E/K(184)E; R(175)Q or H(147)G, with each mutein designated by reference to SEQ ID NO:2.

13. The method according to any one of claims 10 to 12, wherein said KGF includes an initial methionine residue.

14. The method according to any one of claims 1 to 13, wherein the KGF is produced in a prokaryotic cell.

15. The method according to claim 14, wherein the prokaryotic cell is *E. coli.*

16. The method according to any one of claims 1 to 13, wherein the KGF is produced in a mammalian cell.

17. The method according to claim 16, wherein the eukaryotic cell is a Chinese hamster ovary cell.

18. The method according to claim 15, wherein the method comprises:
a) obtaining a solution comprising ΔN23, wherein the initial methionine is optional;
b) loading the solution of step a) onto a cation exchange resin selected from carboxymethyl cellulose, carboxymethyl agarose, sulfated agarose or cellulose columns;
c) eluting the KGF in an eluate solution from the cation exchange resin;
d) contacting the eluate solution with an oxidizing agent, wherein the oxidizing agent is selected from cystamine dihydrochloride, cystine, oxidized glutathione or divalent copper;
e) passing the eluate solution of step d) through a hydrophobic interaction chromatography matrix selected from an alkyl-or phenyl-substituted resin;
and
f) recovering ΔN23 from the hydrophobic interaction chromatography matrix.

## Patentansprüche

1. Verfahren zum Reinigen eines Keratinozyten-Wachstumsfaktors (KGF), wobei das Verfahren die folgenden Schritte umfasst:
a) Herstellen einer Lösung, die den KGF enthält;
b) Auftragen der Lösung von Schritt a) auf ein Kationenaustauscherharz;
c) Eluieren des KGF in einer Eluatlösung aus dem Kationenaustauscherharz;
d) Passieren der Eluatlösung von Schritt c) entweder (i) durch eine Molekulargewichtausschluss-Matrix oder (ii) durch eine hydrophobe-Wechselwirkung-Chromatographie-Matrix; und
e) Gewinnen des KGF,
mit der Maßgabe, dass das Verfahren keinen Schritt einer Heparin-Affinitätschromatographie umfasst.

2. Verfahren nach Anspruch 1, wobei das Kationenaustauscherharz ausgewählt ist aus einer Carboxymethylcellulose-, Carboxymethylagarose-, sulfatierten Agarose- oder Cellulose-Säule.

3. Verfahren nach einem der Ansprüche 1 oder 2, wobei die Eluatlösung von Schritt c) durch eine Molekulargewichtausschluss-Matrix passiert wird.

4. Verfahren nach Anspruch 3, wobei die Molekulargewichtausschluss-Matrix ausgewählt ist aus Größenausschluss-Säulen, die auf Agarose, Acrylamid, Silica oder Polymer basieren.

5. Verfahren nach einem der Ansprüche 1 oder 2, wobei die Eluatlösung von Schritt c) durch eine hydrophobe-Wechselwirkung-Chromatographie-Matrix passiert wird.

6. Verfahren nach Anspruch 5, wobei freie Sulfhydrylgruppen des KGF in der Eluatlösung vor dem Passieren der Eluatlösung durch die hydrophobe-Wechselwirkung-Chromatographie-Matrix oxidiert werden.

7. Verfahren nach Anspruch 6, wobei der KGF oxidiert wird, indem er mit einem Oxidationsmittel in Kontakt gebracht wird.

8. Verfahren nach Anspruch 7, wobei das Oxidationsmittel ausgewählt ist aus Cystamin-Dihydrochlorid, Cystin, oxidiertem Glutathion oder zweiwertigem Kupfer.

9. Verfahren nach einem der Ansprüche 1, 2 und 5 bis 8, wobei die hydrophobe Wechselwirkung-Chromatographie-Matrix ausgewählt ist aus einer Säule mit einem Alkyl- oder Phenyl-substituierten Harz.

10. Verfahren nach einem der Ansprüche 1 bis 9, wobei der KGF die Aminosäurereste 32 bis 194 von SEQ ID NO: 2, gegebenenfalls zusammen mit einer Signalsequenz, oder ein Mutein davon umfasst.

11. Verfahren nach Anspruch 10, wobei das Mutein des Keratinozyten-Wachstumsfaktors ausgewählt ist aus:
a) einem Polypeptid, bei dem Reste, die dem Cystein an der Aminosäureposition 32 von SEQ ID NO: 2 und dem Cystein an der Aminosäureposition 46 von SEQ ID NO: 2 entsprechen, ersetzt oder deletiert sind;
b) einem Polypeptid mit veränderter Ladung, in dem einer oder mehrere der Aminosäurereste 72 bis 185 von SEQ ID NO: 2 deletiert oder mit einem neutralen Rest oder einem negativ-geladenen Rest substituiert sind, der so ausgewählt ist, dass ein Protein mit einer reduzierten positiven Ladung erhalten wird;
c) einem Polypeptid, das durch Substituieren von mindestens einer Aminosäure mit einem höheren schleifenbildenden Potential für mindestens eine Aminosäure innerhalb der schleifenbildenden Region von Asn¹⁴⁶-His¹⁴⁷-Tyr¹⁴⁸-Asn¹⁴⁹-Thr¹⁵⁰ von SEQ ID NO: 2 erzeugt wird; oder
d) einem Polypeptid, das eine oder mehrere Aminosäure-Substitutionen, Deletionen oder Additionen innerhalb der Aminosäurereste 154 bis 164 von SEQ ID NO: 2 aufweist.

12. Verfahren nach Anspruch 11, wobei das Mutein ausgewählt ist aus C(32,46)S, ΔN15, ΔN16, ΔN17, ΔN18, ΔN19, ΔN20, ΔN21, ΔN22, ΔN23, ΔN24, ΔN3/C(46)S, ΔN3/C(46)-, ΔN8/C(46)S, ΔN8/C(46)-, C(32,46)S/R(175)E, C(32,46)S/R(175)Q, ΔN23/R(175)Q, C(32,46,71)S, C(32,46,133)S, C(32,46,133,137)S, ΔN23/N(168)E, ΔN23/K(170)E, ΔN23/K(170)Q, ΔN23/R(175)A, ΔN23/R(175)E, ΔN23/R(175)L, ΔN23/K(178)E, ΔN23/K(178)Q, ΔN23(184)E, ΔN23/K(184)Q, ΔN23/Q(183)E/K(184)E; R(175)Q oder H(147)G, wobei jedes Mutein mit Bezug auf die SEQ ID NO: 2 bezeichnet ist.

13. Verfahren nach einem der Ansprüche 10 bis 12, wobei der KGF am Anfang einen Methioninrest umfasst.

14. Verfahren nach einem der Ansprüche 1 bis 13, wobei der KGF in einer prokaryotischen Zelle hergestellt wird.

15. Verfahren nach Anspruch 14, wobei die prokaryotische Zelle E. coli ist.

16. Verfahren nach einem der Ansprüche 1 bis 13, wobei der KGF in einer Säugerzelle hergestellt wird.

17. Verfahren nach Anspruch 16, wobei die eukaryotische Zelle eine Ovarzelle des Chinesischen Hamsters ist.

18. Verfahren nach Anspruch 15, wobei das Verfahren Folgendes umfasst:
a) Herstellen einer Lösung, die ΔN23 enthält, wobei der am Anfang stehende Methioninrest fakultativ ist;
b) Auftragen der Lösung von Schritt a) auf ein Kationenaustauscherharz, das ausgewählt ist aus Säulen mit Carboxymethylcellulose, Carboxymethylagarose, sulfatierter Agarose oder Cellulose;
c) Eluieren des KGF in einer Eluatlösung aus dem Kationenaustauscherharz;
d) Inkontaktbringen der Eluatlösung mit einem Oxidationsmittel, wobei das Oxidationsmittel ausgewählt ist aus Cystamin-Dihydrochlorid, Cystin, oxidiertem Glutathion oder zweiwertigem Kupfer;
e) Passieren der Eluatlösung von Schritt d) durch eine hydrophobe-Wechselwirkung-Chromatographie-Matrix, die ausgewählt ist aus einem Alkyloder Phenyl-substituierten Harz; und
f) Gewinnen von ΔN23 aus der hydrophobe-Wechselwirkung-Chromatographie-Matrix.

## Revendications

1. Procédé pour purifier un facteur de croissance des kératinocytes (KGF), ledit procédé comprenant les étapes suivantes :
a) obtenir une solution comprenant le KGF ;
b) charger la solution de l'étape a) sur une résine échangeuse de cations ;
c) éluer le KGF dans un éluat à partir de la résine échangeuse de cations ;
d) passer l'éluat de l'étape c) à travers soit (i) une matrice d'exclusion de poids moléculaire ou (ii) une matrice chromatographique à interaction hydrophobe ; et
e) récupérer le KGF,
à condition que ledit procédé ne comprenne pas d'étape de chromatographie d'affinité sur colonne d'héparine.

2. Procédé selon la revendication 1, dans lequel la résine échangeuse de cations est choisie parmi une colonne à carboxyméthyl-cellulose, à carboxyméthyl-agarose, à agarose sulfatée ou à cellulose.

3. Procédé selon l'une quelconque des revendications 1 et 2, dans lequel l'éluat de l'étape c) est passé à travers une matrice d'exclusion de poids moléculaire.

4. Procédé selon la revendication 3, dans lequel la matrice d'exclusion de poids moléculaire est choisie parmi des colonnes d'exclusion stérique à base d'agarose, d'acrylamide, de silice ou de polymères.

5. Procédé selon l'une quelconque des revendications 1 ou 2, dans lequel l'éluat de l'étape c) est passé à travers une matrice chromatographique à interaction hydrophobe.

6. Procédé selon la revendication 5, dans lequel les groupements sulfhydryle libres du KGF de l'éluat sont oxydés préalablement au passage de l'éluat à travers la matrice chromatographique à interaction hydrophobe.

7. Procédé selon la revendication 6, dans lequel le KGF est oxydé en étant mis au contact d'un agent oxydant.

8. Procédé selon la revendication 7, dans lequel l'agent oxydant est choisi parmi du dihydrochlorure de cystamine, de la cystine, du glutathion oxydé ou du cuivre divalent.

9. Procédé selon l'une quelconque des revendications 1, 2 et 5 à 8, dans lequel la matrice chromatographique à interaction hydrophobe est choisie parmi une colonne ayant une résine substituée par des groupements alkyle ou phényle.

10. Procédé selon l'une quelconque des revendications 1 à 9, dans lequel ledit KGF comprend les résidus d'acides aminés 32-194 de SEQ ID No : 2, avec éventuellement une séquence signal, ou une mutéine de ceux-ci.

11. Procédé selon la revendication 10, dans lequel ladite mutéine du facteur de croissance des kératinocytes est choisie parmi
a) un polypeptide dans lequel des résidus correspondant à la cystéine en position d'acide aminé 32 de SEQ ID No : 2 et la cystéine en position d'acide aminé 46 de SEQ ID No : 2 sont remplacées ou délétées ;
b) un polypeptide à changement de charge dans lequel un ou plusieurs des résidus d'acides aminés 72-185 de SEQ ID No : 2 sont délétés ou substitués par un résidu neutre ou un résidu chargé négativement sélectionné de manière à obtenir un protéine avec une charge positive réduite ;
c) un polypeptide généré par la substitution d'au moins un acide aminé ayant un potentiel de formation de boucle plus élevé pour au moins un acide aminé dans la région de formation de boucle de Asn¹⁴⁶-His¹⁴⁷-Tyr¹⁴⁸-Asn¹⁴⁹-Thr¹⁵⁰ de SEQ ID No : 2 ; ou
d) un polypeptide ayant une ou plusieurs substitutions, délétions ou additions d'acides aminés dans les résidus d'acides aminés 154-164 de SEQ ID No : 2 .

12. Procédé selon la revendication 11, dans lequel. ladite mutéine est choisie parmi C(32,46)S, ΔN15, ΔN16, ΔN17, ΔN18, ΔN19, ΔN20, ΔN21, ΔN22, ΔN23, ΔN24, ΔN3/C(46)S, ΔN3/C(46)-, ΔN8/C(46)S, ΔN8/C(46)-, C(32,96)S/R(175)E, C(32,46)S/R(175)Q, ΔN23/R(175)Q, C(32,46,71)S, C(32,46,133)S, C(32,46,133,137)S, ΔN23/N(168)E, ΔN23/K(170)E, ΔN23/K(170)Q, ΔN23/R(175)A, ΔN23/R(175)E, ΔN23/R(175)L, ΔN23/K(178)E, ΔN23/K(178)Q, ΔN23(184)E, ΔN23/K(184)Q, ΔN23/Q(183)E/K(184)E ; R(175)Q ou H(147)G, chaque mutéine étant désignée en référence à SEQ ID No : 2.

13. Procédé selon l'une quelconque des revendications 10 à 12, dans lequel ledit KGF comprend un résidu de méthionine initiale.

14. Procédé selon l'une quelconque des revendications 1 à 13, dans lequel le KGF est produit dans une cellule procaryote.

15. Procédé selon la revendication 14, dans lequel la cellule procaryote est *E*. *coli.*

16. Procédé selon l'une quelconque des revendications 1 à 13, dans lequel le KGF est produit dans une cellule de mammifère.

17. Procédé selon la revendication 16, dans lequel la cellule eucaryote est une cellule d'ovaire de hamster chinois.

18. Procédé selon la revendication 15, dans lequel le procédé comprend :
a) obtenir une solution comprenant ΔN23, dans laquelle la méthionine initiale est facultative ;
b) charger la solution de l'étape a) sur une résine échangeuse de cations choisie parmi des colonnes à carboxyméthyl-cellulose, à carboxyméthyl-agarose, à agarose sulfatée ou à cellulose ;
c) éluer le KGF dans un éluat à partir de la résine échangeuse de cations ;
d) mettre l'éluat en contact avec un agent oxydant, l'agent oxydant étant choisi parmi du dihydrochlorure de cystamine, de la cystine, du glutathion oxydé ou du cuivre divalent ;
e) passer l'éluat de l'étape d) à travers une matrice chromatographique à interaction hydrophobe choisie parmi une résine substituée par des groupements alkyle ou phényle ;
et
f) récupérer ΔN23 à partir de la matrice chromatographique à interaction hydrophobe.
